# FASCICULE DE BREVET EUROPEEN

(11) **EP 1 974 035 B1**
(45) Date de publication et mention de la délivrance du brevet: **14.12.2016**
(21) Numéro de dépôt: 06847097.0
(22) Date de dépôt: 21.12.2006
(51) Int. Cl.: C12N 15/11, A61K 31/7105

(54) **ARN INTERFERENTS CIBLANT LE GENE DE LA NUCLEOPROTEINE DE MORBILLIVIRUS**
INTERFERIERENDE RNA GEGEN DAS NUCLEOPROTEIN EINES MORBILLIVIURS
INTERFERING RNAS TARGETING THE MORBILLIVIRUS NUCLEOPROTEIN GENE

(30) Priorité: 21.12.2005 FR 0513029; 26.10.2006 FR 0609400
(43) Date de publication de la demande: 01.10.2008
(73) Titulaire: Centre de Cooperation Internationale en Recherche Agronomique pour le Developpement, 75016 Paris (FR)
(72) Inventeur: ALBINA, Emmanuel, 34730 Prades-le-lez (FR); LIBEAU, Geneviève, 34090 Montpellier (FR); KEITA, Djénéba, 34090 Montpellier (FR); SERVAN DE ALMEIDA, Renata, 34270 Claret (FR)
(74) Mandataire: Gevers & Orès
(86) Numéro de dépôt international: PCT/FR2006/002819
(87) Numéro de publication internationale: WO 2007/077339

(56) Documents cités:
- WO-A-2004/045543
- REUTER THORSTEN ET AL: "RNA interference with measles virus N, P, and L mRNAs efficiently prevents and with matrix protein mRNA enhances viral transcription" JOURNAL OF VIROLOGY, vol. 80, no. 12, juin 2006 (2006-06), pages 5951-5957, XP002402876 ISSN: 0022-538X
- DATABASE REGISTRY [Online] 21 juin 2004 (2004-06-21), XP002433591 extrait de STN
- DATABASE REGISTRY [Online] 5 août 2004 (2004-08-05), XP002433590 extrait de STN
- DATABASE REGISTRY [Online] 20 octobre 2004 (2004-10-20), XP002433592 extrait de STN
- DATABASE REGISTRY [Online] 14 décembre 2004 (2004-12-14), XP002433593 extrait de STN
- DATABASE REGISTRY [Online] 15 décembre 2004 (2004-12-15), XP002433594 extrait de STN
- DATABASE REGISTRY [Online] 5 janvier 2005 (2005-01-05), XP002433595 extrait de STN
- DATABASE REGISTRY [Online] 2 mars 2005 (2005-03-02), XP002433596 extrait de STN
- KOSCHEL K ET AL: "MEASLES VIRUS ANTISENSE SEQUENCES SPECIFICALLY CURE CELLS PERSISTENTLY INFECTED WITH MEASIES VIRUS" VIROLOGY, ACADEMIC PRESS,ORLANDO, US, vol. 207, no. 1, 20 février 1995 (1995-02-20), pages 168-178, XP000575957 ISSN: 0042-6822

### SEQUENCE LISTING

<110> CENTRE DE COOPERATION INTERNATIONALE EN RECHERCHE
   AGRONOMIQUE POUR LE DEVELOPPEMENT (CIRAD)
   ALBINA , Emmanuel
   LIBEAU , Geneviève
   KEITA , Djénéba
   SERVAN DE ALMEIDA, Renata
<120> ARN INTERFERENTS CIBLANT LE GENE DE LA NUCLEOPROTEINE DE MORBILLIVIRUS
<130> MJP/mad-F1367-10 PCT
<150> FR 05/13029
   <151> 2005-12-21
<150> FR 06/09400
   <151> 2006-10-26
<160> 138
<170> PatentIn version 3.3
<210> 1
   <211> 18
   <212> RNA
   <213> Artificial sequence
<220>
   <223> séquence consensus
<220>
   <221> misc_feature
   <223> v= g, a ou c ; r = a ou g ; y = c ou u ; w = a ou u ; n = a, c, g ou u
<220>
   <221> misc_feature
   <222> (5)..(6)
   <223> n is a, c, g, or u
<220>
   <221> misc_feature
   <222> (8)..(8)
   <223> n is a, c, g, or u
<220>
   <221> misc_feature
   <222> (14)..(14)
   <223> n is a, c, g, or u
<400> 1
   vrwynnrnug guungrra 18
<210> 2
   <211> 18
   <212> RNA
   <213> Artificial sequence
<220>
   <223> séquence consensus
<220>
   <221> misc_feature
   <223> r = a ou g ; y = c ou u ; w = a ou u ; h = a, c ou u ; n = a, c, g ou u
<220>
   <221> misc_feature
   <222> (5)..(6)
   <223> n is a, c, g, or u
<400> 2
   rrwynnrhug guuhgara 18
<210> 3
   <211> 19
   <212> RNA
   <213> Artificial sequence
<220>
   <223> séquence consensus
<220>
   <221> misc_feature
   <223> v= g, a ou c ; r = a ou g ; y = c ou u ; w = a ou u ; n = a, c, g ou u
<220>
   <221> misc_feature
   <222> (6)..(7)
   <223> n is a, c, g, or u
<220>
   <221> misc_feature
   <222> (9)..(9)
   <223> n is a, c, g, or u
<220>
   <221> misc_feature
   <222> (15)..(15)
   <223> n is a, c, g, or u
<400> 3
   vvrwynnrnu gguungrra 19
<210> 4
   <211> 18
   <212> RNA
   <213> Measles virus
<400> 4
   gguucggaug guucgaga 18
<210> 5
   <211> 18
   <212> RNA
   <213> Rinderpest virus
<400> 5
   agucuuacug guuugaga 18
<210> 6
   <211> 18
   <212> RNA
   <213> Peste-des-petits-ruminants virus
<400> 6
   ggaucaacug guuugaga 18
<210> 7
   <211> 18
   <212> RNA
   <213> canine distemper virus
<400> 7
   aauuaggcug guuagaga 18
<210> 8
   <211> 18
   <212> RNA
   <213> Phocine distemper virus
<400> 8
   aaaugggcug guuagaaa 18
<210> 9
   <211> 18
   <212> RNA
   <213> Dolphin morbillivirus
<400> 9
   gaacccauug guuugaga 18
<210> 10
   <211> 18
   <212> RNA
   <213> Artificial sequence
<220>
   <223> séquence consensus
<220>
   <221> misc_feature
   <223> r = a ou g ; y = c ou u ; w = a ou u ; b = g, u ou c ; n = a, c, g ou u
<220>
   <221> misc_feature
   <222> (5)..(5)
   <223> n is a, c, g, or u
<220>
   <221> misc_feature
   <222> (11)..(11)
   <223> n is a, c, g, or u
<220>
   <221> misc_feature
   <222> (13)..(14)
   <223> n is a, c, g, or u
<400> 10
   uyycnaacca nynnrwyb 18
<210> 11
   <211> 18
   <212> RNA
   <213> Artificial sequence
<220>
   <223> séquence consensus
<220>
   <221> misc_feature
   <223> r = a ou g ; y = c ou u ; w = a ou u ; d = a, g ou u ; h = a, c ou u ; n = a, c, g ou u
<220>
   <221> misc_feature
   <222> (13)..(14)
   <223> n is a, c, g, or u
<400> 11
   uyucdaacca dynnrwyy 18
<210> 12
   <211> 19
   <212> RNA
   <213> Artificial sequence
<220>
   <223> séquence consensus
<220>
   <221> misc_feature
   <223> r = a ou g ; y = c ou u ; w = a ou u ; b = g, u ou c ; n = a, c, g ou u
<220>
   <221> misc_feature
   <222> (5)..(5)
   <223> n is a, c, g, or u
<220>
   <221> misc_feature
   <222> (11)..(11)
   <223> n is a, c, g, or u
<220>
   <221> misc_feature
   <222> (13)..(14)
   <223> n is a, c, g, or u
<400> 12
   uyycnaacca nynnrwybb 19
<210> 13
   <211> 18
   <212> RNA
   <213> Measles virus
<400> 13
   ucucgaacca uccgaacc 18
<210> 14
   <211> 18
   <212> RNA
   <213> Rinderpest virus
<400> 14
   ucucaaacca guaagacu 18
<210> 15
   <211> 18
   <212> RNA
   <213> Peste-des-petits-ruminants virus
<400> 15
   ucucaaacca guugaucc 18
<210> 16
   <211> 18
   <212> RNA
   <213> canine distemper virus
<400> 16
   ucucuaacca gccuaauu 18
<210> 17
   <211> 18
   <212> RNA
   <213> Phocine distemper virus
<400> 17
   uuucuaacca gcccauuu 18
<210> 18
   <211> 18
   <212> RNA
   <213> Dolphin morbillivirus
<400> 18
   ucucaaacca auggguuc 18
<210> 19
   <211> 19
   <212> RNA
   <213> Artificial sequence
<220>
   <223> séquence consensus
<220>
   <221> misc_feature
   <223> r = a ou g ; y = c ou u ; m = a ou c ; k = g ou u ; n = a, c, g ou u ; w = a ou u
<220>
   <221> misc_feature
   <222> (2)..(2)
   <223> n is a, c, g, or u
<220>
   <221> misc_feature
   <222> (14)..(14)
   <223> n is a, c, g, or u
<220>
   <221> misc_feature
   <222> (17)..(17)
   <223> n is a, c, g, or u
<400> 19
   gnmkruuywu ggunkcnyu 19
<210> 20
   <211> 19
   <212> RNA
   <213> Artificial sequence
<220>
   <223> séquence consensus
<220>
   <221> misc_feature
   <223> r = a ou g ; y = c ou u ; m = a ou c ; d = a, g ou u ; v = g, a ou c ; k = g ou u ; s = g ou c
<400> 20
   gsmgruuyau gguvkcdyu 19
<210> 21
   <211> 18
   <212> RNA
   <213> Artificial sequence
<220>
   <223> séquence consensus
<220>
   <221> misc_feature
   <223> r = a ou g ; y = c ou u ; h = a, c ou u ; d = a, g ou u ; n = a, c, g ou u ; s = g ou c
<220>
   <221> misc_feature
   <222> (5)..(5)
   <223> n is a, c, g, or u
<220>
   <221> misc_feature
   <222> (8)..(8)
   <223> n is a, c, g, or u
<400> 21
   chyungsnyu dcaygaru 18
<210> 22
   <211> 19
   <212> RNA
   <213> Artificial sequence
<220>
   <223> séquence consensus
<220>
   <221> misc_feature
   <223> r = a ou g ; y = c ou u ; h = a, c ou u ; d = a, g ou u ; n = a, c, g ou u ; s = g ou c
<220>
   <221> misc_feature
   <222> (5)..(5)
   <223> n is a, c, g, or u
<220>
   <221> misc_feature
   <222> (8)..(8)
   <223> n is a, c, g, or u
<400> 22
   chyungsnyu dcaygaruu 19
<210> 23
   <211> 19
   <212> RNA
   <213> Artificial sequence
<220>
   <223> séquence consensus
<220>
   <221> misc_feature
   <223> r = a ou g ; y = c ou u ; h = a, c ou u ; d = a, g ou u ; n = a, c, g ou u ; s = g ou c
<220>
   <221> misc_feature
   <222> (6)..(6)
   <223> n is a, c, g, or u
<220>
   <221> misc_feature
   <222> (9)..(9)
   <223> n is a, c, g, or u
<400> 23
   gchyungsny udcaygaru 19
<210> 24
   <211> 19
   <212> RNA
   <213> Artificial sequence
<220>
   <223> séquence consensus
<220>
   <221> misc_feature
   <223> r = a ou g ; y = c ou u ; h = a, c ou u ; d = a, g ou u ; n = a, c, g ou u
<220>
   <221> misc_feature
   <222> (9)..(9)
   <223> n is a, c, g, or u
<400> 24
   gchyudggny udcaygaru 19
<210> 25
   <211> 19
   <212> RNA
   <213> Measles virus
<400> 25
   gccgauucau ggucgcucu 19
<210> 26
   <211> 19
   <212> RNA
   <213> Measles virus
<400> 26
   gcucuuggac ugcaugaau 19
<210> 27
   <211> 19
   <212> RNA
   <213> Rinderpest virus
<400> 27
   gcagauuuau gguggcauu 19
<210> 28
   <211> 19
   <212> RNA
   <213> Rinderpest virus
<400> 28
   gcacugggcc ugcaugaau 19
<210> 29
   <211> 19
   <212> RNA
   <213> Peste-des-petits-ruminants virus
<400> 29
   ggcgguucau gguaucucu 19
<210> 30
   <211> 19
   <212> RNA
   <213> Peste-des-petits-ruminants virus
<400> 30
   gcauuaggcc uucacgagu 19
<210> 31
   <211> 19
   <212> RNA
   <213> canine distemper virus
<400> 31
   ggcgauucau gguggcgcu 19
<210> 32
   <211> 19
   <212> RNA
   <213> canine distemper virus
<400> 32
   gcucuugggu ugcaugagu 19
<210> 33
   <211> 19
   <212> RNA
   <213> Phocine distemper virus
<400> 33
   ggcgauuuau gguggcauu 19
<210> 34
   <211> 19
   <212> RNA
   <213> Phocine distemper virus
<400> 34
   gcacuugguc uacaugagu 19
<210> 35
   <211> 19
   <212> RNA
   <213> Dolphin morbillivirus
<400> 35
   ggagauucau gguggcauu 19
<210> 36
   <211> 19
   <212> RNA
   <213> Dolphin morbillivirus
<400> 36
   gccuuagggu ugcaugaau 19
<210> 37
   <211> 19
   <212> RNA
   <213> Artificial sequence
<220>
   <223> séquence consensus
<220>
   <221> misc_feature
   <223> r = a ou g ; y = c ou u ; m = a ou c ; k = g ou u ; s = g ou c ; w = a ou u ; n = a, c, g ou u
<220>
   <221> misc_feature
   <222> (3)..(3)
   <223> n is a, c, g, or u
<220>
   <221> misc_feature
   <222> (6)..(6)
   <223> n is a, c, g, or u
<220>
   <221> misc_feature
   <222> (18)..(18)
   <223> n is a, c, g, or u
<400> 37
   arngmnacca wraaymknc 19
<210> 38
   <211> 19
   <212> RNA
   <213> Artificial sequence
<220>
   <223> séquence consensus
<220>
   <221> misc_feature
   <223> r = a ou g ; y = c ou u ; m = a ou c ; b = g, u ou c ; h = a, c ou u ; k = g ou u ; s = g ou c
<400> 38
   arhgmbacca uraaycksc 19
<210> 39
   <211> 18
   <212> RNA
   <213> Artificial sequence
<220>
   <223> séquence consensus
<220>
   <221> misc_feature
   <223> r = a ou g ; y = c ou u ; d = g, a ou u ; i n = a, c, g ou u ; s = g ou c
<220>
   <221> misc_feature
   <222> (11)..(11)
   <223> n is a, c, g, or u
<220>
   <221> misc_feature
   <222> (14)..(14)
   <223> n is a, c, g, or u
<400> 39
   ayucrughar nscnardg 18
<210> 40
   <211> 19
   <212> RNA
   <213> Artificial sequence
<220>
   <223> séquence consensus
<220>
   <221> misc_feature
   <223> r = a ou g ; y = c ou u ; d = g, a ou u ; n = a, c, g ou u ; s = g ou c
<220>
   <221> misc_feature
   <222> (12)..(12)
   <223> n is a, c, g, or u
<220>
   <221> misc_feature
   <222> (15)..(15)
   <223> n is a, c, g, or u
<400> 40
   aayucrugha rnscnardg 19
<210> 41
   <211> 19
   <212> RNA
   <213> Artificial sequence
<220>
   <223> séquence consensus
<220>
   <221> misc_feature
   <223> r = a ou g ; y = c ou u ; d = g, a ou u ; n = a, c, g ou u ; s = g ou c
<220>
   <221> misc_feature
   <222> (11)..(11)
   <223> n is a, c, g, or u
<220>
   <221> misc_feature
   <222> (14)..(14)
   <223> n is a, c, g, or u
<400> 41
   ayucrughar nscnardgc 19
<210> 42
   <211> 19
   <212> RNA
   <213> Artificial sequence
<220>
   <223> séquence consensus
<220>
   <221> misc_feature
   <223> r = a ou g ; y = c ou u ; d = g, a ou u ; h = a, c ou u ; n = a, c, g ou u
<220>
   <221> misc_feature
   <222> (11)..(11)
   <223> n is a, c, g, or u
<400> 42
   ayucrughar ncchardgc 19
<210> 43
   <211> 19
   <212> RNA
   <213> Measles virus
<400> 43
   agagcgacca ugaaucggc 19
<210> 44
   <211> 19
   <212> RNA
   <213> Rinderpest virus
<400> 44
   aaugccacca uaaaucugc 19
<210> 45
   <211> 19
   <212> RNA
   <213> Peste-des-petits-ruminants virus
<400> 45
   agagauacca ugaaccgcc 19
<210> 46
   <211> 19
   <212> RNA
   <213> canine distemper virus
<400> 46
   agcgccacca ugaaucgcc 19
<210> 47
   <211> 19
   <212> RNA
   <213> Phocine distemper virus
<400> 47
   aaugccacca uaaaucgcc 19
<210> 48
   <211> 19
   <212> DNA
   <213> Dolphin morbillivirus
<400> 48
   aaugccacca ugaaucucc 19
<210> 49
   <211> 19
   <212> RNA
   <213> Measles virus
<400> 49
   auucaugcag uccaagagc 19
<210> 50
   <211> 19
   <212> RNA
   <213> Rinderpest virus
<400> 50
   auucaugcag gcccagugc 19
<210> 51
   <211> 19
   <212> RNA
   <213> Peste-des-petits-ruminants virus
<400> 51
   acucgugaag gccuaaugc 19
<210> 52
   <211> 19
   <212> RNA
   <213> canine distemper virus
<400> 52
   acucaugcaa cccaagagc 19
<210> 53
   <211> 19
   <212> RNA
   <213> Phocine distemper virus
<400> 53
   acucauguag accaagugc 19
<210> 54
   <211> 19
   <212> DNA
   <213> Dolphin morbillivirus
<400> 54
   auucaugcaa cccuaaggc 19
<210> 55
   <211> 21
   <212> DNA
   <213> Artificial sequence
<220>
   <223> Amorce PCR
<400> 55
   gtctcggaaa tcgcctcaca g 21
<210> 56
   <211> 21
   <212> DNA
   <213> Artificial sequence
<220>
   <223> Amorce PCR
<400> 56
   cctcctcctg gtcctccaga a 21
<210> 57
   <211> 21
   <212> DNA
   <213> Peste-des-petits-ruminants virus
<400> 57
   ggaucaacug guuugagaat t 21
<210> 58
   <211> 21
   <212> DNA
   <213> Peste-des-petits-ruminants virus
<400> 58
   uucucaaacc aguugaucct t 21
<210> 59
   <211> 21
   <212> DNA
   <213> Peste-des-petits-ruminants virus
<400> 59
   gcucaccuuc auucuuuuct t 21
<210> 60
   <211> 21
   <212> DNA
   <213> Peste-des-petits-ruminants virus
<400> 60
   gaaaagaaug aaggugagct c 21
<210> 61
   <211> 21
   <212> DNA
   <213> Peste-des-petits-ruminants virus
<400> 61
   ggccaguuuc auucuuacut t 21
<210> 62
   <211> 21
   <212> DNA
   <213> Peste-des-petits-ruminants virus
<400> 62
   aguaagaaug aaacuggcct c 21
<210> 63
   <211> 21
   <212> DNA
   <213> Peste-des-petits-ruminants virus
<400> 63
   gagaacucaa uucagaacat t 21
<210> 64
   <211> 21
   <212> DNA
   <213> Peste-des-petits-ruminants virus
<400> 64
   uguucugaau ugaguucuct t 21
<210> 65
   <211> 21
   <212> DNA
   <213> Peste-des-petits-ruminants virus
<400> 65
   ggcgguucau gguaucucut t 21
<210> 66
   <211> 21
   <212> DNA
   <213> Peste-des-petits-ruminants virus
<400> 66
   agagauacca ugaaccgcct t 21
<210> 67
   <211> 21
   <212> DNA
   <213> Peste-des-petits-ruminants virus
<400> 67
   gcauuaggcc uucacgagut t 21
<210> 68
   <211> 21
   <212> DNA
   <213> Peste-des-petits-ruminants virus
<400> 68
   acucgugaag gccuaaugct t 21
<210> 69
   <211> 21
   <212> DNA
   <213> Peste-des-petits-ruminants virus
<400> 69
   guaucaacag cuaggagagt t 21
<210> 70
   <211> 21
   <212> DNA
   <213> Peste-des-petits-ruminants virus
<400> 70
   cucuccuagc uguugauact t 21
<210> 71
   <211> 21
   <212> DNA
   <213> Peste-des-petits-ruminants virus
<400> 71
   gaacuuuggc aggucauaut t 21
<210> 72
   <211> 21
   <212> DNA
   <213> Peste-des-petits-ruminants virus
<400> 72
   auaugaccug ccaaaguuct t 21
<210> 73
   <211> 21
   <212> DNA
   <213> Peste-des-petits-ruminants virus
<400> 73
   cgccaguuuc auucuuacut t 21
<210> 74
   <211> 21
   <212> DNA
   <213> Peste-des-petits-ruminants virus
<400> 74
   aguaagaaug aaacuggcgt t 21
<210> 75
   <211> 21
   <212> DNA
   <213> Rinderpest virus
<400> 75
   gcagucuuac ugguuugagt t 21
<210> 76
   <211> 21
   <212> DNA
   <213> Rinderpest virus
<400> 76
   cucaaaccag uaagacugct t 21
<210> 77
   <211> 21
   <212> DNA
   <213> Rinderpest virus
<400> 77
   cagucuuacu gguuugagat t 21
<210> 78
   <211> 21
   <212> DNA
   <213> Rinderpest virus
<400> 78
   ucucaaacca guaagacugt c 21
<210> 79
   <211> 21
   <212> DNA
   <213> Rinderpest virus
<400> 79
   gcagauuuau gguggcauut t 21
<210> 80
   <211> 21
   <212> DNA
   <213> Rinderpest virus
<400> 80
   aaugccacca uaaaucugct t 21
<210> 81
   <211> 21
   <212> DNA
   <213> Rinderpest virus
<400> 81
   gcacugggcc ugcaugaaut t 21
<210> 82
   <211> 21
   <212> DNA
   <213> Rinderpest virus
<400> 82
   auucaugcag gcccagugct t 21
<210> 83
   <211> 21
   <212> DNA
   <213> Measles virus
<400> 83
   gguucggaug guucgggaat t 21
<210> 84
   <211> 21
   <212> DNA
   <213> Measles virus
<400> 84
   uucccgaacc auccgaacct t 21
<210> 85
   <211> 21
   <212> DNA
   <213> Artificial sequence
<220>
   <223> siARN-GAPDH sens
<400> 85
   aaggucaucc augacaacut t 21
<210> 86
   <211> 21
   <212> DNA
   <213> Artificial sequence
<220>
   <223> siARN-GAPDH antisens
<400> 86
   aguugucaug gaugaccuut t 21
<210> 87
   <211> 21
   <212> DNA
   <213> Peste-des-petits-ruminants virus
<400> 87
   aaaggaucaa cugguuugat t 21
<210> 88
   <211> 21
   <212> DNA
   <213> Peste-des-petits-ruminants virus
<400> 88
   ucaaaccagu ugauccuuut c 21
<210> 89
   <211> 21
   <212> DNA
   <213> Peste-des-petits-ruminants virus
<400> 89
   aaggaucaac ugguuugagt t 21
<210> 90
   <211> 21
   <212> DNA
   <213> Peste-des-petits-ruminants virus
<400> 90
   cucaaaccag uugauccuut t 21
<210> 91
   <211> 21
   <212> DNA
   <213> Peste-des-petits-ruminants virus
<400> 91
   aggaucaacu gguuugagat t 21
<210> 92
   <211> 21
   <212> DNA
   <213> Peste-des-petits-ruminants virus
<400> 92
   ucucaaacca guugauccut t 21
<210> 93
   <211> 21
   <212> DNA
   <213> Peste-des-petits-ruminants virus
<400> 93
   gaucaacugg uuugagaact t 21
<210> 94
   <211> 21
   <212> DNA
   <213> Peste-des-petits-ruminants virus
<400> 94
   guucucaaac caguugauct t 21
<210> 95
   <211> 21
   <212> DNA
   <213> Peste-des-petits-ruminants virus
<400> 95
   aucaacuggu uugagaacat t 21
<210> 96
   <211> 21
   <212> DNA
   <213> Peste-des-petits-ruminants virus
<400> 96
   uguucucaaa ccaguugaut c 21
<210> 97
   <211> 21
   <212> DNA
   <213> Peste-des-petits-ruminants virus
<400> 97
   ucggcgguuc augguaucut t 21
<210> 98
   <211> 21
   <212> DNA
   <213> Peste-des-petits-ruminants virus
<400> 98
   agauaccaug aaccgccgat t 21
<210> 99
   <211> 21
   <212> DNA
   <213> Peste-des-petits-ruminants virus
<400> 99
   cggcgguuca ugguaucuct t 21
<210> 100
   <211> 21
   <212> DNA
   <213> Peste-des-petits-ruminants virus
<400> 100
   gagauaccau gaaccgccgt t 21
<210> 101
   <211> 21
   <212> DNA
   <213> Peste-des-petits-ruminants virus
<400> 101
   gcgguucaug guaucucuct t 21
<210> 102
   <211> 21
   <212> DNA
   <213> Peste-des-petits-ruminants virus
<400> 102
   gagagauacc augaaccgct t 21
<210> 103
   <211> 21
   <212> DNA
   <213> Peste-des-petits-ruminants virus
<400> 103
   cgguucaugg uaucucucat t 21
<210> 104
   <211> 21
   <212> DNA
   <213> Peste-des-petits-ruminants virus
<400> 104
   ugagagauac caugaaccgt t 21
<210> 105
   <211> 21
   <212> DNA
   <213> Peste-des-petits-ruminants virus
<400> 105
   cugcauuagg ccuucacgat t 21
<210> 106
   <211> 21
   <212> DNA
   <213> Peste-des-petits-ruminants virus
<400> 106
   ucgugaaggc cuaaugcagt t 21
<210> 107
   <211> 21
   <212> DNA
   <213> Peste-des-petits-ruminants virus
<400> 107
   ugcauuaggc cuucacgagt t 21
<210> 108
   <211> 21
   <212> DNA
   <213> Peste-des-petits-ruminants virus
<400> 108
   cucgugaagg ccuaaugcat t 21
<210> 109
   <211> 21
   <212> DNA
   <213> Peste-des-petits-ruminants virus
<400> 109
   cauuaggccu ucacgaguut t 21
<210> 110
   <211> 21
   <212> DNA
   <213> Peste-des-petits-ruminants virus
<400> 110
   aacucgugaa ggccuaaugt t 21
<210> 111
   <211> 21
   <212> DNA
   <213> Peste-des-petits-ruminants virus
<400> 111
   auuaggccuu cacgaguugt t 21
<210> 112
   <211> 21
   <212> DNA
   <213> Peste-des-petits-ruminants virus
<400> 112
   caacucguga aggccuaaut t 21
<210> 113
   <211> 21
   <212> DNA
   <213> Rinderpest virus
<400> 113
   acgcagauuu augguggcat t 21
<210> 114
   <211> 21
   <212> DNA
   <213> Rinderpest virus
<400> 114
   ugccaccaua aaucugcgut t 21
<210> 115
   <211> 21
   <212> DNA
   <213> Rinderpest virus
<400> 115
   cgcagauuua ugguggcaut t 21
<210> 116
   <211> 21
   <212> DNA
   <213> Rinderpest virus
<400> 116
   augccaccau aaaucugcgt t 21
<210> 117
   <211> 21
   <212> DNA
   <213> Rinderpest virus
<400> 117
   cagauuuaug guggcauugt t 21
<210> 118
   <211> 21
   <212> DNA
   <213> Rinderpest virus
<400> 118
   caaugccacc auaaaucugt t 21
<210> 119
   <211> 21
   <212> DNA
   <213> Rinderpest virus
<400> 119
   agauuuaugg uggcauugat t 21
<210> 120
   <211> 21
   <212> DNA
   <213> Rinderpest virus
<400> 120
   ucaaugccac cauaaaucut t 21
<210> 121
   <211> 21
   <212> DNA
   <213> Rinderpest virus
<400> 121
   cagcacuggg ccugcaugat t 21
<210> 122
   <211> 21
   <212> DNA
   <213> Rinderpest virus
<400> 122
   ucaugcaggc ccagugcuct t 21
<210> 123
   <211> 21
   <212> DNA
   <213> Rinderpest virus
<400> 123
   agcacugggc cugcaugaat t 21
<210> 124
   <211> 21
   <212> DNA
   <213> Rinderpest virus
<400> 124
   uucaugcagg cccagugcut t 21
<210> 125
   <211> 21
   <212> DNA
   <213> Rinderpest virus
<400> 125
   cacugggccu gcaugaauut t 21
<210> 126
   <211> 21
   <212> DNA
   <213> Rinderpest virus
<400> 126
   aauucaugca ggcccagugt t 21
<210> 127
   <211> 21
   <212> DNA
   <213> Rinderpest virus
<400> 127
   acugggccug caugaauuct t 21
<210> 128
   <211> 21
   <212> DNA
   <213> Rinderpest virus
<400> 128
   gaauucaugc aggcccagut t 21
<210> 129
   <211> 21
   <212> DNA
   <213> Rinderpest virus
<400> 129
   agucuuacug guuugagaat t 21
<210> 130
   <211> 21
   <212> DNA
   <213> Rinderpest virus
<400> 130
   uucucaaacc aguaagacut c 21
<210> 131
   <211> 21
   <212> DNA
   <213> Rinderpest virus
<400> 131
   gucuuacugg uuugagaaut t 21
<210> 132
   <211> 21
   <212> DNA
   <213> Rinderpest virus
<400> 132
   cccgaaccau ccgaaccugt t 21
<210> 133
   <211> 21
   <212> DNA
   <213> Measles virus
<400> 133
   cagguucgga ugguucgggt t 21
<210> 134
   <211> 21
   <212> DNA
   <213> Measles virus
<400> 134
   uucucaaacc aguaagacut c 21
<210> 135
   <211> 21
   <212> DNA
   <213> Measles virus
<400> 135
   agguucggau gguucgggat t 21
<210> 136
   <211> 21
   <212> DNA
   <213> Measles virus
<400> 136
   ucccgaacca uccgaaccut t 21
<210> 137
   <211> 21
   <212> DNA
   <213> Measles virus
<400> 137
   guucggaugg uucgggaact t 21
<210> 138
   <211> 21
   <212> DNA
   <213> Measles virus
<400> 138
   guucccgaac cauccgaact t 21

## Description

La présente invention est relative à l'obtention d'ARNs interférents capables d'inhiber la réplication de morbillivirus, et à leur utilisation pour la prophylaxie ou la thérapie d'infections à morbillivirus, notamment pour l'obtention de vaccins.

Le genre des *Morbillivirus* appartient à la famille des *Paramyxoviridae* (Ordre des Mononegavirales) et regroupe notamment le PPRV (virus de la peste des petits ruminants), le RPV (Rinderpest virus ou virus de la peste bovine), le MV (ou MeV) (Measles virus ou virus de la rougeole), le CDV (canine distemper virus ou virus de la maladie de Carré), le DMV (dolphin morbillivirus ou morbillivirus du dauphin) et le PDV (phocine distemper virus ou virus du phoque veau marin).

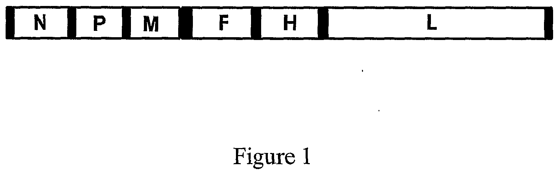

Le génome des morbillivirus est constitué d'un ARN monocaténaire non segmenté, de polarité négative. Sa structure est schématisée sur la Figure 1. Cet ARN monocaténaire comprend 6 gènes : *N, P, M, F, H et L.*

Les produits des gènes *N* (Nucléoprotéine), *P* (Phosphoprotéine) et *L* (protéine Large ; ARN polymérase ARN dépendante) s'associent avec l'ARN génomique viral pour former la nucléocapside, qui protège le génome viral, et constitue un complexe polymérasique permettant la réplication et la transcription du virus.

Les produits des gènes *F* (protéine de Fusion) et *H* (Hémagglutinine) font partie de l'enveloppe virale. La glycoprotéine H permet l'attachement du virus à la cellule cible, et la glycoprotéine F intervient dans la fusion de l'enveloppe virale et de la membrane cellulaire.

Le produit du gène *M* (protéine de Matrice) assure l'interface entre la nucléocapside et l'enveloppe virale.

L'interférence ARN est un mécanisme biologique conservé au cours de l'évolution, qui induit l'extinction spécifique de gènes par dégradation spécifique des ARNs messagers et/ou arrêt de leur traduction. Elle a été observée initialement chez *Caenorhabditis elegans* : l'injection chez ce nématode d'ARN double-brin inhibe l'expression du gène qui contient la séquence de la molécule injectée. Il a ensuite été montré (FIRE et al., Nature, 391, 806, 1998) que c'était l'ARN double-brin qui était à l'origine de ce mécanisme. Dans la cellule, cet ARN double-brin est rapidement segmenté par une endonucléase de type ARNase III, appelée DICER, en petits ARN de 21 à 28 nucléotides : les « small interfering ARN » siARN (ZAMORE et al., Cell, 101, 25, 2000). Les siARNs sont incorporés dans un complexe enzymatique nommé RISC pour « RNA-Induced Silencing Complex ». Le complexe RISC dissocie les brins des siARNs, et guide l'appariement des brins antisens avec leurs séquences-cibles complémentaires. Les ARNs messagers contenant ces séquences-cibles sont alors clivés au niveau du duplex ainsi formé ou leur traduction par les ribosomes est bloquée.

Chez les mammifères, la présence d'ARN double-brin de taille supérieure à 30pb dans une cellule induit également une réponse médiée par l'interféron, qui se traduit par une dégradation des ARN messagers, non spécifique de séquence. Il a été montré (ELBASHIR et al., Nature, 411, 494, 2001) que l'utilisation de siARNs dans des cellules de mammifères n'induisait pas cette réponse de type interféron, et permettait d'inhiber spécifiquement l'expression des gènes contenant les séquences complémentaires de ces siARNs.

L'interférence ARN a été largement utilisée vis-à-vis de virus de diverses familles, pour cibler différents gènes dans le but d'étudier leur fonction, et/ou à des fins de thérapie anti-virale.

En ce qui concerne les virus à ARN monocaténaire négatif non segmenté, les premières expérimentations ont été effectuées chez un pneumovirus, le virus respiratoire syncitial (RSV) (BITKO & BARIK, BMC Microbiol, 1, 34, 2001). Un siARN ciblant le gène de la protéine P (sous-unité de l'ARN polymérase ARN dépendante), a permis d'obtenir une réduction de 90% de l'expression de cette protéine, s'accompagnant d'une diminution drastique de l'expression de l'ensemble des protéines virales, et d'une réduction du titre viral ; un siARN ciblant le gène de la protéine F induit une réduction spécifique de la synthèse de cette protéine, sans affecter celle des autres protéines virales, et inhibe la formation de syncitia.

La Demande PCT WO2005/056021 décrit l'utilisation d'un siARN pour cibler et inactiver le gène codant pour une protéine non-structurelle du RSV, la protéine NS1, et augmenter ainsi la réponse interféron vis-à-vis du RSV.

Une publication récente de S. BARIK (BARIK, Virus Res, 102, 27, 2004) passe en revue les différentes approches utilisées pour contrôler la réplication de virus à ARN monocaténaire négatif non segmenté à l'aide de siARN. Le ciblage des gènes codant les protéines P ou L, qui constituent des sous-unités essentielles du complexe polymérasique viral, conduit à une disparition quasi-totale de la synthèse de l'ARN viral ; le ciblage de gènes non essentiels à la synthèse de l'ARN viral, mais impliqués dans les interactions du virus avec la cellule-hôte produit des résultats plus variables.

L'interférence ARN représente potentiellement un outil particulièrement intéressant pour la prophylaxie et/ou le traitement des infections virales.

Cependant, malgré tout l'intérêt théorique de cette approche, sa mise en pratique pour obtenir une activité antivirale efficace pose différents problèmes, concernant notamment le choix des séquences-cibles utilisées dans les siARNs.

On utilise ici le terme de « gène-cible » pour désigner un gène dont on cherche à obtenir l'extinction, et celui de « séquence-cible », pour désigner une portion de l'ARNm d'un gène-cible reconnue par un siARN particulier.

L'un des problèmes relatifs au choix de séquences-cibles découle de la fréquence des mutations, qui est généralement très élevée chez les virus. Une mutation intervenant dans la séquence-cible d'un siARN peut permettre au virus mutant d'échapper à la reconnaissance. Il est donc souhaitable de choisir une séquence-cible conservée entre différents virus, où des mutations sont moins susceptibles d'apparaître.

D'autre part, même s'il est relativement aisé, pour un gène cible donné, de définir des siARNs permettant d'obtenir une certaine atténuation de l'expression de ce gène, il est beaucoup plus problématique d'obtenir des siARNs permettant de parvenir à un niveau d'extinction du gène-cible suffisant pour inhiber la réplication virale.

Il est en effet connu que l'efficacité de l'interférence ARN peut varier considérablement d'un siARN à l'autre. De très nombreux facteurs apparaissent impliqués dans cette variabilité, relatifs notamment à la séquence-cible elle-même (par exemple la teneur en G/C, la présence de certaines bases à certaines positions), à la position de cette séquence-cible dans le gène ciblé, et à la présence de structures secondaires de l'ARNm pouvant diminuer l'accessibilité de la séquence-cible pour le siARN. Différentes méthodes ont été proposées pour tenter de prédire l'efficacité des siARNs (GILMORE et al., J Drug Target, 12, 315, 2004 ; UI-TEI & SAIGO, Tanpakushitsu Kakusan Koso, 49, 2662, 2004 ; AMARZGUIOUI & PRYDZ, Biochem Biophys Res Commun, 316, 1050, 2004 ; HEALE et al., Nucleic Acids Res, 33, e30, 2005 ; REYNOLDS et al., Nat Biotechnol, 22, 326, 2004 ; ARZIMAN et al., Nucleic Acids Res, 33, W582, 2005 ; HUESKEN et al., Nat Biotechnol, 23, 995,2005).

Cependant, malgré les progrès effectués dans la rationalisation des critères de choix des séquences-cibles optimales, ce choix demeure en grande partie empirique, et ses résultats aléatoires.

Les Inventeurs ont émis l'hypothèse que l'extinction du gène codant pour la protéine N des morbillivirus pourrait permettre d'obtenir l'inhibition de la réplication virale, et ont entrepris de rechercher si cette extinction pouvait être obtenue à l'aide de siARNs ciblant des régions de ce gène conservées entre les morbillivirus.

Ils sont parvenus à définir, dans ces régions conservées, des loci contenant des séquences-cibles permettant de définir des siARNs capables d'inhiber l'expression de la protéine N, cette inhibition induisant une inhibition de la réplication des morbillivirus.

Au sens de la présente invention, on entend par inhibition de l'expression d'un gène, une diminution d'au moins 85%, de préférence au moins 90% du niveau d'expression d'un gène par rapport à son niveau normal. On entend par inhibition de la réplication virale, une diminution d'au moins 95%, de préférence au moins 98% de la quantité de virus par rapport à celle produite dans des conditions normales de réplication.

La présente invention a en conséquence pour objet un procédé pour inhiber la réplication d'un morbillivirus, caractérisé en ce qu'il comprend l'inhibition du gène N d'un morbillivirus, à l'aide d'un ARN interférent ciblant la région de l'ARNm dudit gène contenant un motif comprenant la séquence générale suivante :
VRWYNNRNUGGUUNGRRA (SEQ ID NO: 1),

Notamment, ledit motif peut être défini par l'une des séquences générales suivantes :

### RRWYNNRHUGGUUHGARA (SEQ ID NO: 2) ou

### WRWYNNRNUGGUUNGRRA (SEQ ID NO: 3)

Les séquences générales mentionnées dans le cadre de l'exposé de la présente invention ont été établies à partir de l'alignement de séquences de gènes *N* des morbillivirus PPRV, RPV, MV, CDV, DMV, et PDV.

Ces séquences sont représentées conformément au code IUPAC, à savoir : A = Adénine ; C = Cytosine ; G = Guanine ; U = Uracile ; K = G ou U ; M = A ou C ; R = A ou G; S = G ou C ; Y = C ou U; W = A ou U ; B = G, U ou C ; D = G, A ou U ; H = A ou C ou U; V = G, A ou C ; N = A ou C ou G ou U.

Par exemple, pour inhiber l'expression du gène *N* du virus de la rougeole, on peut choisir un ARN interférent ciblant la région de l'ARNm dudit gène contenant la séquence suivante : GGUUCGGAUGGUUCGAGA (SEQ ID NO: 4) ; pour inhiber l'expression du gène *N* du RPV, on peut choisir un ARN interférent ciblant la région de l'ARNm dudit gène contenant la séquence suivante : AGUCUUACUGGUUUGAGA (SEQ ID NO: 5) ; pour inhiber l'expression du gène *N* du PPRV, on peut choisir un ARN interférent ciblant la région de l'ARNm dudit gène contenant la séquence suivante : GGAUCAACUGGUUUGAGA (SEQ ID NO: 6) ; pour inhiber l'expression du gène *N* du CDV, on peut choisir un ARN interférent ciblant la région de l'ARNm dudit gène contenant la séquence suivante : AAUUAGGCUGGUUAGAGA (SEQ ID NO: 7) ; pour inhiber l'expression du gène *N* du PDV, on peut choisir un ARN interférent ciblant la région de l'ARNm dudit gène contenant la séquence suivante : AAAUGGGCUGGUUAGAAA (SEQ ID NO: 8) ; pour inhiber l'expression du gène *N* du DMV, on peut choisir un ARN interférent ciblant la région de l'ARNm dudit gène contenant la séquence suivante : GAACCCAUUGGUUUGAGA (SEQ ID NO: 9).

Typiquement, ledit ARN interférent comprend une portion de 19 à 29 pb, de préférence de 19 à 23 pb, d'une séquence antisens du gène *N* d'un morbillivirus, ladite portion contenant une séquence antisens de l'une des séquences-cible définies ici.

Ainsi, un ARN interférent ciblant la région de l'ARNm du gène *N* comprenant la séquence SEQ ID NO: 1 comprendra un motif défini par la séquence générale UYYCNAACCANYNNRWYB (SEQ ID NO: 10). Si ledit ARN interférent cible plus particulièrement la région de l'ARNm du gène *N* définie par la séquence SEQ ID NO: 2, il comprendra un motif défini par la séquence générale UYUCDAACCADYNNRWYY (SEQ ID NO: 11) ; s'il cible plus particulièrement la région de l'ARNm du gène *N* définie par la séquence SEQ ID NO: 3, il comprendra un motif défini par la séquence générale UYYCNAACCANYNNRWYBB (SEQ ID NO: 12).

A titre d'exemples, pour cibler la région de l'ARNm du gène *N* comprenant la séquence SEQ ID NO: 1, on peut choisir :
- dans le cas du virus de la rougeole, un ARN interférent qui contient la séquence suivante : UCUCGAACCAUCCGAACC (SEQ ID NO: 13) ;
- dans le cas du RPV, un ARN interférent qui contient la séquence suivante : UCUCAAACCAGUAAGACU (SEQ ID NO: 14) ;
- dans le cas du PPRV, un ARN interférent qui contient la séquence suivante : UCUCAAACCAGUUGAUCC (SEQ ID NO: 15) ;
- dans le cas du CDV, un ARN interférent qui contient la séquence suivante : UCUCUAACCAGCCUAAUU (SEQ ID NO: 16) ;
- dans le cas du PDV, un ARN interférent qui contient la séquence suivante : UUUCUAACCAGCCCAUUU (SEQ ID NO: 17) ;
- dans le cas du DMV, un ARN interférent qui contient la séquence suivante : UCUCAAACCAAUGGGUUC (SEQ ID NO: 18).

Conformément à l'invention, pour inhiber la réplication d'un morbillivirus, on peut également inhiber le gène N dudit morbillivirus à l'aide d'ARN interférents ciblant d'autres régions conservées de l'ARNm dudit gène, à savoir :
- un ARN interférent dirigé contre la région de l'ARNm du gène *N* contenant un motif défini par la séquence générale suivante : GNMKRUUYWUGGUNKCNYU (SEQ ID NO: 19), et avantageusement par la séquence générale suivante : GSMGRUUYAUGGUVKCDYU (SEQ ID NO: 20).
- un ARN interférent dirigé contre la région de l'ARNm du gène *N* contenant un motif comprenant la séquence générale suivante : CHYUNGSNYUDCAYGARU (SEQ ID NO: 21), notamment un motif défini par la séquence générale suivante : CHYUNGSNYUDCAYGARUU (SEQ ID NO: 22), ou un motif défini par la séquence générale suivante : GCHYUNGSNYUDCAYGARU (SEQ ID NO: 23). Avantageusement, ledit motif est défini par la séquence générale suivante : GCHYUDGGNYUDCAYGARU (SEQ ID NO: 24).

A titre d'exemples :
- pour inhiber l'expression du gène *N* du virus de la rougeole, on peut utiliser : un ARN interférent ciblant la région de l'ARNm dudit gène contenant la séquence suivante: GCCGAUUCAUGGUCGCUCU (SEQ ID NO: 25); un ARN interférent ciblant la région de l'ARNm dudit gène contenant la séquence suivante : GCUCUUGGACUGCAUGAAU (SEQ ID NO: 26);
- pour inhiber l'expression du gène *N* du RPV, on peut utiliser : un ARN interférent ciblant la région de l'ARNm dudit gène contenant la séquence suivante : GCAGAUUUAUGGUGGCAUU (SEQ ID NO: 27) ; un ARN interférent ciblant la région de l'ARNm dudit gène contenant la séquence suivante : GCACUGGGCCUGCAUGAAU (SEQ ID NO: 28) ;
- pour inhiber l'expression du gène *N* du PPRV, on peut utiliser : un ARN interférent ciblant la région de l'ARNm dudit gène contenant la séquence suivante : GGCGGUUCAUGGUAUCUCU (SEQ ID NO: 29) ; un ARN interférent ciblant la région de l'ARNm dudit gène contenant la séquence suivante : GCAUUAGGCCUUCACGAGU (SEQ ID NO: 30);
- pour inhiber l'expression du gène N du CDV, on peut utiliser : un ARN interférent ciblant la région de l'ARNm dudit gène contenant la séquence suivante : GGCGAUUCAUGGUGGCGCU (SEQ ID NO: 31) et/ou un ARN interférent ciblant la région de l'ARNm dudit gène contenant la séquence suivante : GCUCUUGGGUUGCAUGAGU (SEQ ID NO: 32) ;
- pour inhiber l'expression du gène *N* du PDV, on peut utiliser : un ARN interférent ciblant la région de l'ARNm dudit gène contenant la séquence suivante : GGCGAUUUAUGGUGGCAUU (SEQ ID NO: 33) ; un ARN interférent ciblant la région de l'ARNm dudit gène contenant la séquence suivante : GCACUUGGUCUACAUGAGU (SEQ ID NO: 34) ;
- pour inhiber l'expression du gène N du DMV, on peut utiliser : un ARN interférent ciblant la région de l'ARNm dudit gène contenant la séquence suivante : GGAGAUUCAUGGUGGCAUU (SEQ ID NO: 35) et/ou un ARN interférent ciblant la région de l'ARNm dudit gène contenant la séquence suivante : GCCUUAGGGUUGCAUGAAU (SEQ ID NO: 36).

Un ARN interférent dirigé contre la région de l'ARNm du gène *N* contenant un motif défini par la séquence SEQ ID NO: 19, comprendra un motif défini par la séquence générale ARNGMNACCAWRAAYMKNC (SEQ ID NO: 37), et avantageusement par la séquence générale ARHGMBACCAURAAYCKSC (SEQ ID NO: 38).

Un ARN interférent dirigé contre une région de l'ARNm du gène *N* comprenant un motif contenant la séquence générale SEQ ID NO: 21, comprendra un motif contenant la séquence générale AYUCRUGHARNSCNARDG (SEQ ID NO: 39). Plus particulièrement, si ledit ARN interférent est dirigé contre la région de l'ARNm du gène N comprenant le motif défini par la séquence SEQ ID NO: 22, il comprendra un motif défini par la séquence générale AAYUCRUGHARNSCNARDG (SEQ ID NO: 40). S'il est dirigé contre la région de l'ARNm du gène N comprenant le motif défini par la séquence SEQ ID NO: 23, il comprendra un motif défini par la séquence générale AYUCRUGHARNSCNARDGC (SEQ ID NO: 41), et avantageusement par la séquence générale AYUCRUGHARNCCHARDGC (SEQ ID NO: 42).

A titre d'exemples, pour cibler la région de l'ARNm du gène *N* comprenant le motif défini par la séquence SEQ ID NO: 19, on peut choisir :
- dans le cas du virus de la rougeole, un ARN interférent contenant la séquence suivante : AGAGCGACCAUGAAUCGGC (SEQ ID NO: 43);
- dans le cas du RPV, un ARN interférent contenant la séquence suivante : AAUGCCACCAUAAAUCUGC (SEQ ID NO: 44) ;
- dans le cas du PPRV, un ARN interférent contenant la séquence suivante : AGAGAUACCAUGAACCGCC (SEQ ID NO: 45) ;
- dans le cas du CDV, un ARN interférent contenant la séquence suivante : AGCGCCACCAUGAAUCGCC (SEQ ID NO: 46) ;
- dans le cas du PDV, un ARN interférent contenant la séquence suivante : AAUGCCACCAUAAAUCGCC (SEQ ID NO: 47) ;
- dans le cas du DMV, un ARN interférent contenant la séquence suivante : AAUGCCACCAUGAAUCUCC (SEQ ID NO: 48).

De même, pour cibler la région de l'ARNm du gène N comprenant le motif défini par la séquence SEQ ID NO: 21, on peut choisir :
- dans le cas du virus de la rougeole, un ARN interférent contenant la séquence suivante : AUUCAUGCAGUCCAAGAGC (SEQ ID NO: 49);
- dans le cas du RPV, un ARN interférent contenant la séquence suivante : AUUCAUGCAGGCCCAGUGC (SEQ ID NO: 50) ;
- dans le cas du PPRV, un ARN interférent contenant la séquence suivante : ACUCGUGAAGGCCUAAUGC (SEQ ID NO: 51) ;
- dans le cas du CDV, un ARN interférent contenant la séquence suivante : ACUCAUGCAACCCAAGAGC (SEQ ID NO: 52) ;
- dans le cas du PDV, un ARN interférent contenant la séquence suivante : ACUCAUGUAGACCAAGUGC (SEQ ID NO: 53) ;
- dans le cas du DMV, un ARN interférent contenant la séquence suivante : AUUCAUGCAACCCUAAGGC (SEQ ID NO: 54).

Pour la mise en oeuvre de la présente invention, on peut utiliser séparément un ARN interférent ciblant la région de l'ARNm du gène N comprenant la séquence SEQ ID NO: 1, un ARN interférent ciblant la région de l'ARNm du gène *N* comprenant la séquence SEQ ID NO: 19, et un ARN interférent ciblant la région de l'ARNm du gène N comprenant la séquence SEQ ID NO: 21. Avantageusement, on peut utiliser une combinaison d'ARN interférents ciblant deux de ces régions, ou une combinaison d'ARN interférents ciblant les trois régions. On peut également, le cas échéant, utiliser un ou plusieurs de ces ARN interférents en combinaison avec un ou plusieurs autres ARN interférents ciblant d'autres régions du génome de morbillivirus.

La présente invention a également pour objet les ARNs interférents utilisables pour la mise en oeuvre du procédé conforme à l'invention, à savoir tout ARN interférent dirigé contre une région du gène N d'un morbillivirus contenant un motif défini par l'une des séquences générales SEQ ID NO: 1, SEQ ID NO: 19, SEQ ID NO: 21, ci-dessus.

Plus précisément, des ARNs interférents conformes à l'invention sont :
- des ARNs interférents comprenant un motif défini par la séquence générale SEQ ID NO: 10, notamment ceux comprenant un motif défini par la séquence générale SEQ ID NO: 11, ou un motif défini par la séquence générale SEQ ID NO: 12. A titre d'exemples particuliers on citera les ARN interférents comprenant un motif défini par l'une des séquences SEQ ID NO: 13 à SEQ ID NO: 18 ;
- des ARNs interférents comprenant un motif défini par la séquence générale SEQ ID NO: 37, et avantageusement par la séquence générale SEQ ID NO: 38. A titre d'exemples particuliers on citera les ARN interférents comprenant un motif défini par l'une des séquences SEQ ID NO: 43 à SEQ ID NO: 48 ;
- des ARNs interférents comprenant un motif défini par la séquence générale SEQ ID NO: 39, notamment ceux comprenant un motif défini par la séquence générale SEQ ID NO: 40, ou par la séquence générale SEQ ID NO: 41, avantageusement par la séquence générale SEQ ID NO: 42. A titre d'exemples particuliers on citera les ARN interférents comprenant un motif défini par l'une des séquences SEQ ID NO: 49 à SEQ ID NO: 54.

Les différents ARNs interférents définis ci-dessus peuvent se présenter sous diverses formes.

Il peut s'agir d'un ARN antisens simple-brin, capable de s'intégrer dans le complexe RISC, et de s'apparier avec la séquence cible, induisant son clivage (TIJSTERMAN et al., Science, 295, 694, 2002 ; MARTINEZ et al., Cell, 110, 563, 2002 ; BARIK, Virus Res, 102, 27, 2004).

Cependant il s'agira de préférence d'un ARN contenant à la fois la séquence-cible et la séquence antisens correspondante, sous forme de siARN, ou le cas échéant de sh(« short hairpin »)ARNs (YU et al., Proc Natl Acad Sci USA, 99, 6047, 2002 ; PADDISON et al., Genes Dev, 16, 948, 2002 ; SIOLAS et al., Nat Biotechnol, 23, 227, 2005), qui est transformé dans la cellule en siARN.

Les siARNs ont généralement une longueur de 21 à 25 nucléotides ; ils contiennent une portion double-brin, généralement de 19 à 21 nucléotides, constituée de la séquence cible et de la séquence antisens correspondante ; l'un ou l'autre brin, ou les deux, comporte(nt) généralement à l'extrémité 3' une extension simple-brin de 2 ou 3 nucléotides ; le plus souvent (mais non obligatoirement), il s'agit d'un dinucléotide, de séquence TT.

Les shARNs sont constitués d'un seul brin, d'une longueur de 50 à 70 nucléotides, qui peut se replier pour former une structure en épingle à cheveux, contenant une portion double-brin d'une longueur de 19 à 29 nucléotides, constituée par la séquence cible et la séquence antisens correspondante, et une boucle de 5 à 10 nucléotides. Ils peuvent comprendre également, à l'extrémité 3', une extension simple-brin de 2 ou 3 nucléotides.

Les ARN interférents peuvent être aussi utilisés sous forme de précurseurs de microARNs (pre-miARN). Les microARNs (miARN) sont des ARNs d'environ 22 nucléotides, constitués d'un seul brin et qui se fixent sur l'extrémité 3' non codante de l'ARNm, ce qui a pour effet de réprimer l'expression de cet ARNm sans le dégrader (contrairement aux siARNs ou shARNs). Les miARNs sont des molécules importantes pour la régulation « naturelle » de l'expression des gènes dans les cellules eucaryotes. Les miARNs sont produits dans le cytoplasme des cellules à partir de précurseurs nucléaires d'environ 70 nucléotides en forme d'épingle à cheveux, appelés pre-miARNs, clivés en miARNs par le complexe DICER. La séquence d'un pre-miARN, identifié dans les conditions naturelles dans une cellule eucaryote, peut être modifiée pour y introduire une séquence siARN, qui, libérée par le DICER ira alors dégrader un autre ARNm. Ce pre-miARN modifié sert alors de « véhicule » pour le siARN d'intérêt, ce qui a pour résultat d'augmenter l'efficacité de l'interférence. La possibilité d'utiliser des pre-miARNs modifiés pour inhiber la réplication virale a été démontrée par BODEN et al. (Nucleic Acids Res., 13, 1154, 2004) pour HIV-1. Dans ce cas, l'efficacité de l'interférence peut être supérieure de 80% à celle conférée par le siARN équivalent.

Ces ARNs interférents peuvent être obtenus par des méthodes classiques de préparation des acides nucléiques (pour revue cf. par exemple AMARZGUIOUI et al., FEBS Lett, 579, 5974, 2005).

Ils peuvent ainsi être préparés par exemple par synthèse chimique, ou bien par génie génétique. Dans ce dernier cas on utilisera un vecteur d'expression contenant une séquence d'ADN pouvant être transcrite en un ARN interférent conforme à l'invention, placée sous contrôle d'un promoteur approprié. Généralement, ledit promoteur est un promoteur viral, par exemple le promoteur T3, T7, SP6, pCMV ou un promoteur reconnu par la polymérase III, par exemple le promoteur du petit ARN *U6,* ou celui de l'ARN *H1* (MIYAGISHI & TAIRA, Nucleic Acids Res Suppl, 113, 2002); des promoteurs reconnus par la polymérase II sont toutefois également utilisables.

Les vecteurs d'expression définis ci-dessus font également partie de l'objet de la présente invention.

De très nombreuses méthodes pour introduire des ARNs interférents dans des cellules ou des organismes dans lesquelles on souhaite obtenir l'extinction de l'expression d'un gène-cible sont connues en elles-mêmes.

Dans le cas où l'on souhaite obtenir une extinction temporaire de l'expression du gène-cible, on peut administrer directement l'ARN interférent, préférablement associé à un véhicule approprié, permettant de faciliter son entrée dans la cellule et/ou de le protéger de la dégradation. A titre d'exemples de véhicules utilisables, on citera notamment des liposomes ou des nanoparticules.

Pour obtenir une extinction à plus long terme de l'expression du gène-cible, notamment dans le cas des cellules de mammifères, on utilise un vecteur permettant d'exprimer l'ARN interférent dans la cellule. De très nombreux vecteurs utilisables dans ce but sont connus en eux-mêmes. On citera notamment des vecteurs dérivés de rétrovirus, de lentivirus, ou d'adénovirus (BARTON & MEDZHITOV, Proc Natl Acad Sci USA, 99, 14943, 2002 ; TISCORNIA et al., Proc Natl Acad Sci USA, 100, 1844, 2003; XIA et al., Nat Biotechnol, 20, 1006, 2002 ; SHEN et al., FEBS Lett, 539, 111,2003).

La présente invention a également pour objet l'utilisation d'un ARN interférent ou d'un vecteur d'expression conforme à l'invention pour l'obtention d'un médicament destiné au traitement ou à la prévention d'une infection à morbillivirus, et notamment au traitement ou à la prévention de la rougeole, de la peste bovine, de la peste des petits ruminants, de la maladie de Carré, de la peste des phoques ou d'une infection au DMV.

La présente invention a également pour objet une composition pharmaceutique comprenant un ARN interférent ou un vecteur d'expression conforme à l'invention. Avantageusement, ladite composition pharmaceutique est un vaccin.

La présente invention sera mieux comprise à l'aide du complément de description qui va suivre, qui se réfère à des exemples illustrant l'obtention d'un ARN interférent conforme à l'invention, et son utilisation pour bloquer la réplication des morbillivirus PPRV et RPV.

### LEGENDE DES FIGURES

**Figure 1** **:** Représentation schématique du génome des morbillivirus.
**Figure 2** **:** Effet des siARNs sur l'effet cytopathique (ECP) induit par le virus de la peste des petits ruminants sur des cellules VERO. Ordonnées : échelle de pourcentages d'inhibition de l'ECP. Abscisses : concentration des siARNs en nM.
   --?-- : siARN NPPR1 ; --?-- : siARN NPPR2 ; --?-- : siARN NPPR3 ; --?-- siARN NPPR10 ; --|-- :siARN GAPDH, cellules non-infectées; --|-- : cellules infectées non transfectées.
**Figure 3** **:** Effet des siARNs sur l'expression de la protéine N du virus PPRV dans les cellules VERO. (a) Abscisses : fluorescence liée à l'expression de la protéine N ; Ordonnées : nombre de cellules fluorescentes ; siARN 1 : siARN NPPR1 ; siARN2 : siARN NPPR2 ; cellules non infectées : contrôle négatif ; cellules infectées : contrôle positif. (b) Abscisses : concentration (en nM) des siARNs. Ordonnées : pourcentage de cellules fluorescentes ;
   ? : siARN NPPR1 ; | : siARN GAPDH.
**Figure 4** **:** Effet des siARNs NPPR1, 2 et 3 sur la synthèse d'ARN viraux mesurée par RT-PCR quantitative en temps réel (QRT-PCR). Représentation de deux essais indépendants.
**Figure 5** **:** Effet des siARNs NPPR1 (hachures verticales), NPPR2 (blanc), NPPR3 (grisé), NPPR10 (noir) et GAPDH (hachures diagonales) sur la réplication virale mesurée par le titre viral dans les tapis cellulaires infectés.
**Figure 6** **:** Effet des siARNs sur l'expression des protéines N de PPRV, RPV et MV dans les cellules VERO.(a)pourcentage d'inhibition de la protéine N de PPRV en fonction des loci NPPR1-3 à NPPR1+2, NPPR6-2 à NPPR6+2, NPPR7-2 à NPPR7+2 ; (b) pourcentage d'inhibition de la protéine N de PRV en fonction des loci NRP1, NRP1+1 à NRP1+3, NRP6-2 à NRP6+2 et NRP7-2 à NRP7+2, (c) pourcentage d'inhibition de la protéine N de MV en fonction des locus NMV1-2 à NMV1+1.

### EXEMPLES : MATERIEL ET METHODES

### 1) Virus et séquences d'intérêt

Le virus PPRV utilisé est la souche Nigeria 75/1 (DIALLO et al. Rev Elev Med Vet Pays Trop 42, 311, 1989), atténuée par passages en série sur cellules (SK/1, BK/1 et Vero/55). Il s'agit d'une souche vaccinale. La séquence complète du génome de cette souche est disponible sur Genbank (numéro d'accession X74443).

Le virus RPV utilisé est la souche RBOK (PLOWRIGHT et FERRIS, Res. Vet. Sci, 3, 172, 1962), souche vaccinale à virus atténué par passages en série sur cellules (BK/98 et Vero/2). La séquence complète du génome de cette souche est disponible sur Genbank (numéro d'accession Z30697).

Le virus MV utilisé est la souche Schwarz La séquence complète du génome de cette souche est disponible sur Genbank (MVU03668).

Les virus PPRV, RPV et MV sont multipliés sur cellules VERO (ATCC) entretenues en monocouche en présence de milieu complet soit : milieu essentiel de Eagle MEM avec sels de Earle (Eurobio, Courtaboeuf, France), 10% de sérum de foetus de bovin (Eurobio, Courtaboeuf, France) et 2 mM de L-glutamine (Gibco, Mife Technologies, UK).

### 2) Culture cellulaire, transfection et infection virale

Des cellules VERO adhérentes sont décollées à l'aide d'une solution contenant de la trypsine et de l'EDTA (Sigma-Aldrich, Lyon, France) puis resuspendués dans du milieu MEM (Minimum Eagle Medium) complet à raison de 10⁵ cellules/ ml et distribuées dans des puits de plaque 24 puits. Les plaques sont incubées à 37°C et 5% CO₂. Par la suite, toutes les incubations de cellules ont lieu à 37°C en présence de 5% de CO₂. Lorsque le tapis cellulaire atteint 70-80% de confluence, le milieu est éliminé, puis les cellules sont incubées 30 minutes dans du milieu MEM dépourvu de sérum de foetus de bovin.

Pour la transfection, le milieu est éliminé et remplacé par de la LIPOFECTAMINE™ 2000 (Invitrogen) à raison de 500 ng dans 200 µl de milieu de transfection OPTI-MEM I® Invitrogen) contenant les siRNA à différentes concentrations (de 6,5 à 100 nM final dans le mélange de transfection). L'incubation suivante a lieu pendant trois heures.

Pour l'infection virale, le milieu de transfection est éliminé et remplacé par du MEM et 5% de sérum de foetus de bovin. Les cellules sont incubées 24 heures puis infectées avec les virus MV, PPRV ou RPV à une multiplicité d'infection de 0,1 dose cytopathique 50% par cellule, en milieu sans sérum de foetus de bovin, la dose cytopathique 50% étant la quantité de virus induisant un ECP sur 50% des tapis cellulaires infectés. Après une heure de contact, le tapis cellulaire est rincé deux fois avec du milieu MEM, puis du milieu MEM avec 5% de sérum de foetus de bovin est ajouté sur les cellules. Les cellules sont observées quotidiennement et l'évolution de l'effet cytopathique (ECP) dû au virus est évaluée selon une grille de pourcentages (échelle allant de 0%, pas d'ECP à 100%, ECP maximal). L'effet des siARN est exprimé en pourcentage d'inhibition de l'ECP.
Quatre à cinq jours après infection, le surnageant de culture cellulaire d'une part, et les cellules d'autre part sont recueillis pour analyse en titrage viral et en expression d'antigènes viraux par cytométrie en flux. Des contrôles consistant en des cellules non transfectées et non infectées et des cellules infectées et non transfectées sont inclus dans chaque série de tests des siARNs.

### 4) Titrage viral

Les surnageants de culture cellulaire ou les cellules elles-mêmes sont conservés à -70°C jusqu'à utilisation pour titrage. Le titrage viral est effectué sur culture cellulaire (VERO) à partir d'une série de dilutions de raison 10 de la suspension virale à titrer. Le titre est déterminé selon la méthode de REED et MUENCH (Am J Trop Med Hyg, 127, 493, 1938) et exprimé en dose cytopathique 50 (DCP₅₀) par ml.

### 5) Mesure de l'expression d'antigènes viraux

L'expression des protéines de nucleocapside (N), de matrice (M) et de fusion (F) virales a été mesurée par cytométrie en flux en utilisant des anticorps monoclonaux spécifiques : anticorps 38-4 spécifique de la protéine N du PPRV ; anticorps IVB2-4 spécifique de la protéine N du RPV; anticorps MAB8906 (CHEMICON INTERNATIONAL) spécifiques de la protéine N de MV, anticorps 19-6 spécifique de la protéine M du PPRV et de la protéine M du RPV (LIBEAU et al., Revue d'Elevage et de Médecine Vétérinaire des Pays Tropicaux, 50, 181-190, 1997), anticorps MAB 128-1 spécifiques de la protéine F de RPV (LIBEAU et al, Revue d'Elevage et de Médecine Vétérinaire des Pays Tropicaux, 50, 181-190, 1997) et anticorps 11/295/33 spécifique de la molécule CD8 lymphocytaire de porc (SAALMULLER et al., Vet Immunol Immunopathol, 43, 249, 1994) utilisé comme contrôle isotypique de spécificité du marquage.

Les cellules adhérentes sont décollées du plastique par incubation avec une solution contenant de la trypsine et de l'EDTA pendant 5 minutes à 37°C. Les cellules sont lavées dans du tampon phosphate PBS, 0,1% d'azide de sodium, 5% de sérum de cheval et 0,0062% de saponine (poids/volume). Elles sont dénombrées et distribuées à raison de 10⁶ cellules par puits d'une plaque 96 puits. Tous les marquages sont effectués en plaque. Après sédimentation des cellules par centrifugation et élimination du surnageant, 100 µl d'une dilution appropriée de l'anticorps monoclonal anti-N, anti-M ou isotypique sont ajoutés et les cellules re-suspendues par agitation mécanique. Les cellules sont incubées 30 minutes à +4°C. Elles sont ensuite lavées deux fois dans le même tampon phosphate, puis incubées 30 minutes à +4°C, dans 50 µl d'une dilution appropriée d'un anticorps anti-anticorps de souris, conjugué à la fluorescéine (BIORAD, France). Les cellules sont lavées deux fois puis fixées 15 minutes à température ambiante avec 100 µl d'une solution de paraformaldéhyde. Les cellules sont resuspendues dans 400 µl de PBS FACS FLOW (Becton Dickinson, USA). L'analyse des cellules s'effectue au FACsort (Becton Dickinson, USA). Les débris cellulaires sont éliminés de l'analyse au FACS par un fenêtrage placé sur FSCxSSC (FSC : Forward SCatter ; SSC : Side SCatter), puis la fluorescence est mesurée sur 20 000 cellules.

### 6) Mesure quantitative de la synthèse des ARN viraux

Les cellules et le surnageant de culture sont récoltés 96 heures après infection et congelés à -70°C. L'ARN total est extrait à partir de 100 µl de cette suspension cellulaire mélangés à la solution de lyse du kit RNeasy, selon les instructions du fabricant (Qiagen, Courtaboeuf, France). La suite du protocole correspond au manuel d'instruction du fabricant. L'ARN extrait est conservé à -70°C. L'ARN est quantifié par QRT-PCR en une étape (Brilliant SYBR Green QRT-PCR Master Mix, 1 step, Stratagene). Les amorces utilisées sont NP3bis = 5'- GTCTCGGAAATCGCCTCACAG-3' (sens) (SEQ ID NO: 55) et NP4bis = 5'- CCTCCTCCTGGTCCTCCAGAA-3' (antisens) (SEQ ID NO: 56). Ces amorces ont été dérivées des amorces précédemment décrites (COUACY-HYMANN et al., J Virol Methods, 100, 17, 2002) par addition d'un G en 5' de NP3bis et délétion de quatre bases en 3' et délétion de trois bases en 3' de NP4bis. Ces amorces amplifient un fragment de 351 bases. Une solution réactionnelle principale constituée de 12,5 µl SYBR Green Master mix (2x), 2,5 µl de 1 µM de chaque amorce, 0,0625 µl de Stratascript Reverse Transcriptase RT et 2,4375 µl d'eau est déposée dans un microtube, puis 5 µl de l'ARN extrait sont ajoutés. La QRT-PCR est mise en oeuvre sur un appareil Mx3000P (Stratagene, Amsterdam, NL) selon le protocole suivant :
- transcription inverse à 50°C pendant 30 minutes
- dénaturation à 95°C pendant 10 minutes
- amplification avec 35 cycles [95°C, 30 secondes ; 55°C, 1 minute ; 72°C, 30 secondes]
- dissociation avec un cycle [95°C, 1 minute ; 55°C, 30 secondes ; 95°C, 30 secondes]

L'étalon standard pour la quantification est constitué du gène de la protéine N du PPRV inséré dans un plasmide pBluescript KS+ (COUACY-HYMANN et al., 2002, précité). Pour la quantification, le plasmide est linéarisé, purifié par précipitation à l'alcool et dilué de façon sérielle de 10 en 10. La quantité d'ARN détectée dans les échantillons soumis à analyse est ramenée au nombre de copies de gène N incorporées dans la série de dilutions de l'étalon standard.

### EXEMPLE 1 : SELECTION ET SYNTHESE DE siARNs

Les zones conservées du gène *N* ont été localisées par un alignement multiple des séquences correspondantes issues de différents morbillivirus à l'aide d'un logiciel d'analyse de séquences (Vector NTI package, Informax Inc). Cet alignement multiple de séquences a été effectué sur les séquences des gènes N des morbillivirus dont les numéros d'accession GenBank sont les suivants :
- PPRV : X74443, AY560591,
- pour RPV : AF378110, Z30697, E06018, X98291,
- pour PDV : X75717,
- pour DMY : AJ608288,
- pour CDV : AF014953, AF164967, AF166268, AF166269, AF166270, AF166271, AF166272, AF166273, AF378705, AY238607, AY241572, AY241573, AY241574, AY241575, AY241576, AY241577, AY241578, AY241579, AY241580, AY241581, AY241582, AY278994, AY278995, AY386315, AY386316, AY390348, AY443350, AY445077, AY466011, AY542312, AY684629, AY738624, AY738625, AY738653, AJ009656, DQ003302, DQ005126, DQ005127, DQ005128, DQ005129, DQ005130, DQ005131, DQ005132, DQ005133, DQ005134, DQ435615, DQ522030, DQ887066, DQ887333, NC_001921, X02000,
- pour MV : AB012948, AB012949, AB016162, AB032167, AB046218, AB052821, AB254456, AF045205, AF045206, AF045207, AF045208, AF045209, AF045210, AF045211, AF045212, AF045213, AF045214, AF045215, AF045216, AF045217, AF045218, AF079555, AF171232, AF243852, AF266286, AF266287, AF266288, AF266289, AF266290, AF266291, AF280800, AF280801, AF280802, AF280803, AF280804, AF417265, AF417266, AF417267, AF417268, AF417269, AF417270, AF417271, AF419319, AF440685, AF440686, AF440687, AF504045, AF504046, AF504047, AF504048, AF504049, AY027637, AY027638, AY027639, AY027640, AY027641, AY027642, AY027643, AY027644, AY184217, AY486083, AY486084, AY556539, AY556540, AY556541, AY556542, AY730614, DQ011612, DQ011613, DQ190373, DQ211902, DQ227318, DQ227319, DQ227320, DQ227321, DQ345721, DQ345723, X16566, X16567, X16568, X16569, D63927, M89921, M89922, L46728, L46730, L46733, L46735, L46740, L46742, L36042, L36046, L46744, L46746, L46748, L46750, L46753, L46756, L46758, L46760, L46764, L46767, Z66517, AJ232191, AJ232192, AJ232193, AJ232194, AJ232195, AJ232196, AJ232197, AJ232198, AJ232199, AJ232200, AJ232201, AJ232202, AJ232203, AJ232204, AJ232205, AJ232206, AJ232207, AJ232208, AJ232209, AJ232210, AJ232211, AJ232212, AJ232213, AJ232214, AJ232215, AJ232216, AJ232217, AJ232218, AJ232219, AJ232220, AJ232221, AJ232770, AJ232771, AJ232772, AJ232773, AJ232774, AJ232775, AJ232776, AJ232777, AJ232778, AJ232779, AJ232780, AJ232781, AJ232782, AJ244027, AJ244028, AJ244029, AJ244030, AJ244031, AJ244032, AJ244033, AJ244034, AJ244035, AJ244036, AJ244037, AJ244038, AJ244039, AJ244040, AJ244041, AJ250068, AJ250069, AJ250070, AJ250071, AJ250072, AJ250073, U01974, U01976, U01977, U01978, U01987, U01988, U01989, U01990, U01991, U01992, U01993, U01994, U01995, U01996, U01998, U01999, U03650, U03653, U03656, U03658, U03661, K01711, U03664, U03668, U29317, U97350, AF266287, X01999, X13480, D10550, S58435

Dans les régions présentant le plus fort degré d'homologie, 7 séquences-cibles ont été définies pour le virus PPRV.

Les siARNs dérivés du gène N du virus PPRV, et contenant des séquences identiques aux séquences-cibles définies dans ce gène, sont dénommés NPPR1, et NPPR5 à 10 ; un siARN dénommé NPPR2, ne présentant au mieux que 58% d'identité avec la séquence du gène *N* du virus PPRV (en positions 256-274, 751-769, et 850-869 de la séquence d'ADNc), et un siARN dénommé NPPR3, dérivé de la même séquence cible que le siARN NPPR10, mais présentant une différence d'une base avec ladite séquence-cible (C dans la séquence cible remplacé par G à l'extrémité 5' du brin sens du siRNA NPPR3) ont également été synthétisés.

Pour le virus RPV, 4 séquences cibles ont été définies au niveau des loci correspondant aux séquences-cible du siARN-NPPR1, siARN-NPPR6 et siARN-NPPR7. Les siARNs correspondants sont dénommés NRP1, NRP1+1, NRP6 et NRP7. NRP1+1 correspond à NRP1 décalé d'une base vers l'extrémité 3'.

Pour le virus MV, 1 séquence cible a été définie au niveau du locus correspondant à la séquence-cible du siARN-NPPR1. Le siARN correspondant est dénommé NM1.

Tous les siARNs mentionnés ci-dessus ont été synthétisés chimiquement par la société Ambion (Europe) (Cambridgeshire, UK).

Pour tester la spécificité de l'interférence, un siARN ciblant la séquence de la Glyceraldehyde-3-phosphate deshydrogenase (GAPDH) a été utilisé : la séquence et le siARN sont disponibles chez Ambion (Europe) (Silencer^{™} GAPDH siARN (Human) Control 4605).

Toutes les séquences siARNs identifiées ont été soumises à une recherche d'homologie dans Genbank à l'aide du logiciel Blast. Le maximum d'identité observé entre le siARN-GAPDH et la séquence codant pour la protéine N du virus PPRV est de 72%.

Les séquences des brins sens et antisens des différents siARNs mentionnés ci-dessus, ainsi que leur position par rapport à la séquence d'ADNc du gène *N* et par rapport à la séquence du cadre de lecture dudit gène, sont indiquées dans le Tableau I ci-après.

**TABLEAU I**

| Nom du siARN | Séquence (SEQ ID #) | Position dans l'ADNc / dans le cadre de lecture |
|---|---|---|
| siARN-NPPR1 | sens : 5'-GGAUCAACUGGUUUGAGAAtt-3' (57) | 480-498/428-446 |
| | antisens : 5'-UUCUCAAACCAGUUGAUCCtt-3' (58) | |
| | | |
| siARN-NPPR2 | sens : 5'-GCUCACCUUCAUUCUUUUCtt-3' (59) | -/- |
| | antisens : 5'-GAAAAGAAUGAAGGUGAGCtc-3' (60) | |
| | | |
| siARN-NPPR3 | sens : 5'-GGCCAGUUUCAUUCUUACUtt-3' (61) | 850-868/798-816 |
| | antisens : 5'-AGUAAGAAUGAAACUGGCCtc-3' (62) | |
| | | |
| siARN-NPPR5 | sens: 5'-GAGAACUCAAUUCAGAACAtt-3' (63) | 1001-1019/949-968 |
| | antisens : 5'-UGUUCUGAAUUGAGUUCUCtt-3' (64) | |
| | | |
| siARN-NPPR6 | sens : 5'-GGCGGUUCAUGGUAUCUCUtt-3' (65) | 741-759/689-707 |
| | antisens : 5'-AGAGAUACCAUGAACCGCCtt-3' (66) | |
| | | |
| siARN-NPPR7 | sens : 5'-GCAUUAGGCCUUCACGAGUtt-3' (67) | 899-917/847-865 |
| | antisens : 5'-ACUCGUGAAGGCCUAAUGCtt-3' (68) | |
| | | |
| siARN-NPPR8 | sens : 5'-GUAUCAACAGCUAGGAGAGtt-3' (69) | 958-976/906-924 |
| | antisens : 5'-CUCUCCUAGCUGUUGAUACtt-3' (70) | |
| | | |
| siARN-NPPR9 | sens:5'-GAACUUUGGCAGGUCAUAUtt-3' (71) | 1102-1120/1050-1068 |
| | antisens : 5'-AUAUGACCUGCCAAAGUUCtt-3' (72) | |
| | | |
| siARN-NPPR10 | sens : 5'-CGCCAGUUUCAUUCUUACUtt-3' (73) | 850-868/798-816 |
| | antisens : 3'-AGUAAGAAUGAAACUGGCGtt-3' (74) | |
| | | |
| siARN-NRP1 | sens : 5'-GCAGUCUUACUGGUUUGAGtt-3' (75) | 478-496/426-444 |
| | antisens : 5'-CUCAAACCAGUAAGACUGCtt-3' (76) | |
| | | |
| siARN-NRP1+1 | sens : 5'-CAGUCUUACUGGUUUGAGAtt-3' (77) | 479-497/427-445 |
| | antisens : 5'-UCUCAAACCAGUAAGACUGtc-3' (78) | |
| | | |
| siARN-NRP6 | sens : 5'-GCAGAUUUAUGGUGGCAUUtt-3' (79) | 741-759/689-707 |
| | antisens : 5'-AAUGCCACCAUAAAUCUGCtt-3' (80) | |
| | | |
| siARN-NRP7 | sens : 5'-GCACUGGGCCUGCAUGAAUtt-3' (81) | 899-917/847-865 |
| | antisens : 5'-AUUCAUGCAGGCCCAGUGCtt-3' (82) | |
| | | |
| siARN-NM1 | sens : 5'-GGUUCGGAUGGUUCGGGAAtt-3' (83) | 480-498/428-446 |
| | antisens : 5'-UUCCCGAACCAUCCGAACCtt-3' (84) | |
| | | |
| siARN-GAPDH | sens : 5'-AAGGUCAUCCAUGACAACUtt-3' (85) | -/- |
| | antisens : 5'-AGUUGUCAUGGAUGACCUUtt-3' (86) | |

### EXEMPLE 2 : EFFET DES siARNs NPPR1, 2, ET 3 ET DU siARN-GAPDH SUR L'EFFET CYTOPATHIQUE DU VIRUS DE LA PESTE DES PETITS RUMINANTS.

Des cellules VERO ont été transfectées avec différentes doses des siARNs NPPR1, 2, et 3 ou du siARN-GAPDH, puis infectées par le PPRV, selon le protocole décrit dans la section « Matériel et Méthodes » ; l'effet cytopathique a été évalué 4 jours après infection, indépendamment par deux personnes différentes.

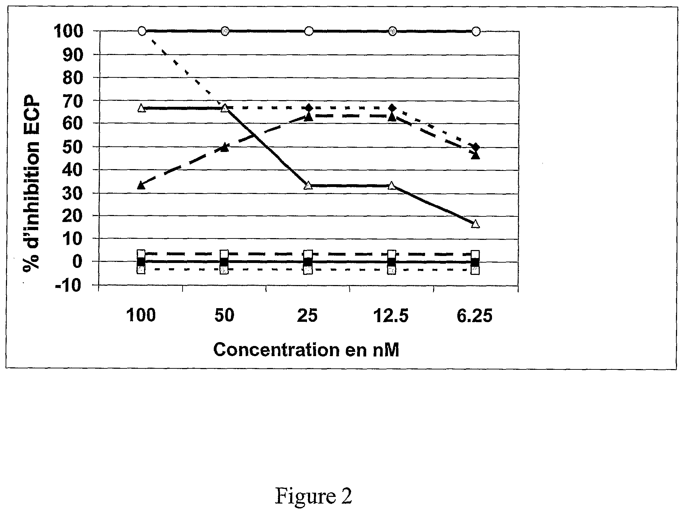

La figure 2 illustre les résultats observés :
Parmi les siARNs testés, le siARN NPPR1 est celui qui inhibe le plus l'ECP, quelle que soit la dose utilisée. A la dose de 100 nM, on observe une inhibition complète de l'ECP. Le siARN NPPR3, dont la séquence varie d'un nucléotide par rapport à la séquence cible définie sur le virus (cf. Exemple 1) inhibe l'ECP de façon moins marquée que NPPR1. Le siARN NPPR10, qui est dirigé contre la même séquence-cible que le siARN NPPR3, mais qui, contrairement à ce dernier est parfaitement complémentaire de ladite séquence-cible, inhibe également l'ECP de façon moins marquée que NPPR1 (résultats non-montrés)
Comme attendu, le siARN-NPPR2 ainsi que le siARN-GAPDH ne produisent aucune neutralisation de l'ECP.

### EXEMPLE 3 : EFFET DES siARN SUR L'EXPRESSION DE LA PROTEINE N ET DE LA PROTEINE M DU PPRV ET DU RPV.

Des cellules VERO ont été transfectées avec différentes doses du siARN NPPR1, du siARN NPPR2 ou du siARN GAPDH, puis infectées par le PPRV, comme décrit dans la section « Matériel et Méthodes » ; 4 à 5 jours après infection, la production de la protéine N dans les cellules est mesurée par cytométrie en flux, comme décrit dans la section « Matériel et Méthodes ».

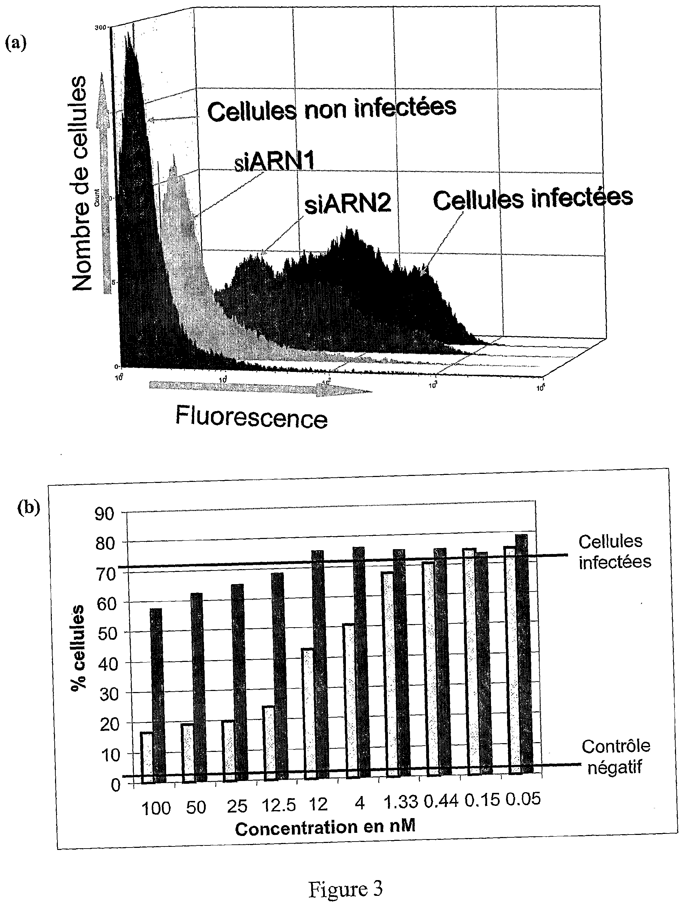

La figure 3 (a) illustre les résultats observés dans des cellules transfectées avec 100 nM du siARN NPPR1 ou du siARN NPPR2, ainsi que dans des cellules non-infectées et non transfectées, et dans des cellules non-transfectées et infectées, utilisées à titre de contrôle. On observe une très forte inhibition de l'expression de la protéine N dans les cellules infectées transfectées avec le siARN NPPR1, alors que dans celles transfectées avec le siARN NPPR2, le profil d'expression de la protéine N est très similaire à celui observé avec les cellules infectées non-transfectées, bien qu'une certaine diminution de l'expression soit observée.

La figure 3 (b) illustre les résultats observés dans des cellules transfectées avec différentes doses du siARN NPPR1 ou du siARN GAPDH. On observe une diminution très significative du pourcentage de cellules exprimant la protéine N à partir de 12,5 nM de siARN NPPR1.

Dans une seconde série d'expérimentations, l'expression de la protéine N et de la protéine M a été mesurée :
- dans des cellules VERO transfectées avec la dose optimale pour chacun des siARNs testés, à savoir 100 nM pour les siARNs NPPR1, 2, 3, ou 5 à 9, ou 25 nM pour le siARN NPPR10, puis infectées avec PPRV ou avec RPV;
- dans des cellules VERO transfectées avec 100 nM de l'un des siARNs NRP1 ou NRP1+1, puis infectées avec PPRV ou avec RPV.

La mesure de l'expression de la protéine M permet de vérifier que l'inhibition de l'expression de la protéine N entraine une inhibition des autres protéines virales, ce qui traduit un effet sur la réplication du génome viral.

Les résultats d'une expérimentation sont résumés par le Tableau II ci-après.

**TABLEAU II**

| Nature du siARN (concentration optimale) | Inhibition de l'expression de la nucléoprotéine PPRV (en %) | Inhibition de l'expression de la protéine de matrice PPRV (en %) | Inhibition de l'expression de la nucléoprotéine RPV (en %) | Inhibition de l'expression de la protéine de matrice RPV (en %) |
|---|---|---|---|---|
| siARN-NPPR1 (100 nM) | 90 | 89 | 0 | 0 |
| siARN-NPPR2 (100 nM) | 25 | 11 | nt | nt |
| siARN-NPPR3 (100 nM) | 40 | 30 | nt | nt |
| siARN-NPPR5 (100 nM) | 16 | nt | nt | nt |
| siARN-NPPR6 (100 nM) | 68 | nt | nt | nt |
| siARN-NPPR7 (100 nM) | 63 | nt | nt | nt |
| siARN-NPPR8 (100 nM) | 34 | nt | nt | nt |
| siARN-NPPR9 (100 nM) | 35 | nt | nt | nt |
| siARN-NPPR10 (25 nM) | 60 | 55 | nt | nt |
| siARN-NRP1 (100 nM) | 0 | nt | 35 | 31 |
| siARN-NRP1+1 (100 nM) | 0 | nt | 97 | 92,5 |
| siARN-GAPDH (100 nM) | 0 | 5 | 13 | 3 |

| | | | | |
|---|---|---|---|---|
| nt : non testé | | | | |

Dans cette expérimentation, quatre des siARN testés (siARNs NPPR1, 6, 7 et 10) ont un effet significatif sur le PPRV. Parmi ceux-ci, seul le siARN NPPR1 permet d'atteindre un pourcentage de 90% d'inhibition de l'expression de la protéine N (les autres siRNAs ne permettent pas d'atteindre 70% d'inhibition de l'expression) et un pourcentage de 89% d'inhibition de l'expression de la protéine M.

En ce qui concerne le RPV, seul le siARN NRP1+1 possède un effet significatif Le siARN NRP1, qui ne contient pas la totalité de la séquence cible, a un effet très amoindri.

Ces résultats montrent que le locus reconnu par les siARNS NPPR1, et NRP1+1 constitue une cible particulièrement intéressante pour l'extinction du gène de la protéine N des morbillivirus. Cette cible est très circonscrite, puisqu'une base manquante diminue considérablement les effets obtenus.

### EXEMPLE 4 : EFFET INHIBITEUR DU siARN NPPR1 SUR LA SYNTHESE DES ARN VIRAUX.

L'effet du siARN NPPR1 sur la réplication virale a été évalué en quantifiant la synthèse de l'ARN viral, comme décrit dans la section «Matériel et Méthodes », dans des cellules VERO infectées par PPRV, non-transfectées, ou transfectées avec 100 nM de l'un des siARNs NPPR1, NPPR3, NPPR2 ou GAPDH, ainsi que dans des cellules VERO non-infectées.

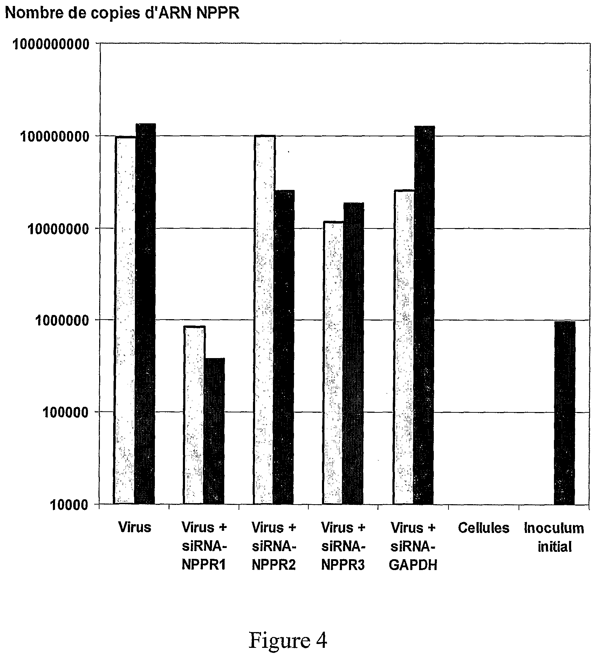

Les résultats sont illustrés par la figure 4.

Dans les cellules infectées non-transfectées, la réplication virale se traduit par une quantité d'ARN viral 100 fois supérieure à celle apportée par l'inoculum initial. La transfection avec les siARNs NPPR2 et GAPDH n'a pas d'effet significatif sur la réplication virale. Dans les cellules transfectées avec NPPR3, la quantité d'ARN viral demeure plus de 10 fois supérieure à celle apportée par l'inoculum initial. En revanche, dans les cellules transfectées avec NPPR1, la quantité d'ARN viral correspond à celle apportée par l'inoculum initial, ce qui confirme que ce siARN inhibe la réplication virale.

### EXEMPLE 5 : EFFET INHIBITEUR DU siARN NPPR1 SUR LA REPLICATION VIRALE MESUREE PAR LE TITRE VIRAL

L'effet du siARN NPPR1 sur la réplication virale a été évalué en mesurant le titre viral, comme décrit dans « Matériels et Méthodes », 4 jours après l'infection par PPRV de cellules VERO transfectées avec 6,25, 12,5, 25, 50 ou 100 nM de l'un des siARNs NPPR1, NPPR2, NPPR3, NPPR10 ou GAPDH.

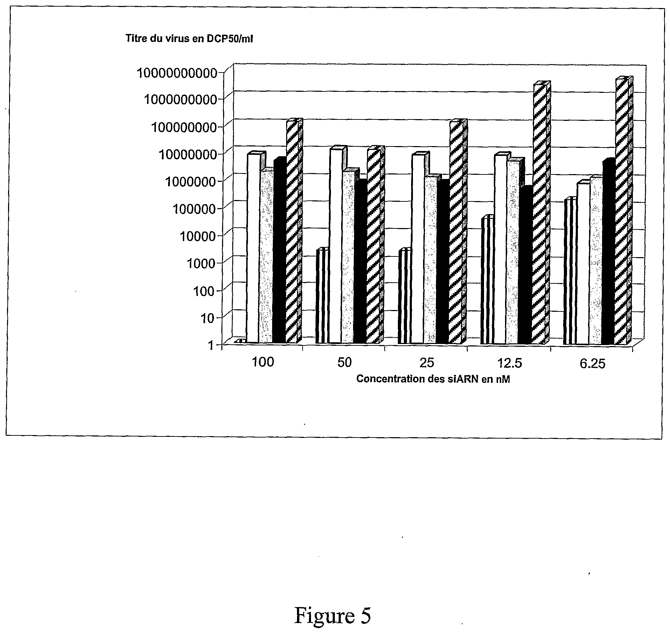

Les résultats sont illustrés par la figure 5.

Pour les cellules transfectées par l'un des siARNs NPPR2, NPPR3 et NPPR10, on observe une légère diminution du titre viral par rapport au contrôle (cellules transfectées par le siARN GAPDH), qui ne varie pas significativement en fonction de la concentration testée.

Pour les cellules transfectées par NPPR1, le titre viral diminue très significativement à partir de 12,5 nM de siARN. A 100 nM de siARN, on observe une inhibition totale de la réplication virale.

### EXEMPLE 6 : DELIMITATION DES LOCI EFFICACES DANS LES GENES N DE PPRV, RPV ET MV.

Pour définir la localisation exacte des loci les plus efficaces, des siARN ont été définis par décalage d'une (+1), deux (+2), ou trois (+3) bases en 3' ou une (-1), deux (-2), ou trois (-3) bases en 5' par rapport aux loci définis dans l'exemple 1 (cf. tableau I).

Pour le virus PPRV les siARN testés sont :
- NPPR1, NPPR1-3, NPPR1-2, NPPR1-1, NPPR1+1 et NPPR1+2,
- NPPR6, NPPR6-2, NPPR6-1, NPPR6+1 et NPPR6+2,
- NPPR7, NPPR7-2, NPPR7-1, NPPR7+1 et NPPR7+2.

Pour le virus RPV les siARN testés sont :
- NRP1, NRP1+1, NRP1+2, NRP1+3
- NRP6, NRP6-2, NRP6-1, NRP6+1 et NRP6+2,
- NRP7, NRP7-2, NRP7-1, NRP7+1 et NRP7+2.

Pour le virus MV les siARN testés sont NM1, NM1-2, NM1-1 et NM1+1.

Les séquences des brins sens et antisens des différents siARNs mentionnés ci-dessus, ainsi que leur position par rapport à la séquence du gène *N* et par rapport à la séquence du cadre de lecture dudit gène, sont indiquées dans le Tableau III ci-après.

**TABLEAU III**

| Nom du siARN | SEQUENCE (SEQ ID #) | Position dans l'ADNc/dans le cadre de lecture |
|---|---|---|
| siARN-NPPR1-3 | sens : 5'-AAAGGAUCAACUGGUUUGAtt-3' (87) | 477-495/425-443 |
| | antisens : 5'-UCAAACCAGUUGAUCCUUUtc-3' (88) | |
| | | |
| siARN-NPPR1-2 | sens : 5'-AAGGAUCAACUGGUUUGAGtt-3' (89) | 478-496/426-444 |
| | antisens : 5'-CUCAAACCAGUUGAUCCUUtt- 3' (90) | |
| | | |
| siARN-NPPR1-1 | sens : 5'-AGGAUCAACUGGUUUGAGAtt-3' (91) | 479-497/427-445 |
| | antisens : 5'-UCUCAAACCAGUUGAUCCUtt-3' (92) | |
| | | |
| siARN-NPPR1+1 | sens : 5'-GAUCAACUGGUUUGAGAACtt-3' (93) | 481-499/429-447 |
| | antisens : 5'-GUUCUCAAACCAGUUGAUCtt-3' (94) | |
| | | |
| siARN-NPPR1+2 | sens : 5'-AUCAACUGGUUUGAGAACAtt-3' (95) | 482-500/430-448 |
| | antisens : 5'-UGUUCUCAAACCAGUUGAUtc-3' (96) | |
| | | |
| siARN-NPPR6-2 | sens : 5'-UCGGCGGUUCAUGGUAUCUtt-3' (97) | 739-757/687-705 |
| | antisens : 5'-AGAUACCAUGAACCGCCGAtt-3' (98) | |
| | | |
| siARN-NPPR6-1 | sens : 5'-CGGCGGUUCAUGGUAUCUCtt-3' (99) | 740-758/688-706 |
| | antisens : 5'-GAGAUACCAUGAACCGCCGtt-3' (100) | |
| | | |
| siARN-NPPR6+1 | sens : 5'-GCGGUUCAUGGUAUCUCUCtt-3' (101) | 742-760/690-708 |
| | antisens : 5'-GAGAGAUACCAUGAACCGCtt-3' (102) | |
| | | |
| siAR-NPPR6+2 | sens : 5'-CGGUUCAUGGUAUCUCUCAtt-3' (103) | 743-761/691-709 |
| | antisens : 5'-UGAGAGAUACCAUGAACCGtt-3' (104) | |
| | | |
| siARN-NPPR7-2 | sens : 5-CUGCAUUAGGCCUUCACGAtt-3' (105) | 897-915/845-863 |
| | antisens : 5'-UCGUGAAGGCCUAAUGCAGtt-3' (106) | |
| | | |
| siARN-NPPR7-1 | sens : 5'-UGCAUUAGGCCUUCACGAGtt-3' (107) | 898-916/846-864 |
| | antisens : 5'-CUCGUGAAGGCCUAAUGCAtt-3' (108) | |
| | | |
| siARN-NPPR7+1 | sens : 5'-CAUUAGGCCUUCACGAGUUtt-3' (109) | 900-918/848-866 |
| | antisens : 5'-AACUCGUGAAGGCCUAAUGtt-3' (110) | |
| | | |
| siARN-NPPR7+2 | sens : 5'-AUUAGGCCUUCACGAGUUGtt-3' (111) | 901-919/849-867 |
| | antisens : 5'-CAACUCGUGAAGGCCUAAUtt-3' (112) | |
| siARN-NRP6-2 | sens : 5'-ACGCAGAUUUAUGGUGGCAtt-3' (113) | 739-757/687-705 |
| | antisens : 5'-UGCCACCAUAAAUCUGCGUtt-3' (114) | |
| | | |
| siARN-NRP6-1 | sens : 5'-CGCAGAUUUAUGGUGGCAUtt-3' (115) | 740-758/688-706 |
| | antisens : 5'-AUGCCACCAUAAAUCUGCGtt-3' (116) | |
| | | |
| siARN-NRP6+1 | sens : 5'-CAGAUUUAUGGUGGCAUUGtt-3' (117) | 742-760/690-708 |
| | antisens : 5'-CAAUGCCACCAUAAAUCUGtt-3' (118) | |
| | | |
| siARN-NRP6+2 | sens : 5'-AGAUUUAUGGUGGCAUUGAtt-3' (119) | 743-761/691-709 |
| | antisens : 5'-UCAAUGCCACCAUAAAUCUtt-3' (120) | |
| | | |
| siARN-NRP7-2 | sens : 5'-CAGCACUGGGCCUGCAUGAtt-3' (121) | 897-915/845-863 |
| | antisens : 5'-UCAUGCAGGCCCAGUGCUCtt-3' (122) | |
| | | |
| siARN-NRP7-1 | sens : 5'-AGCACUGGGCCUGCAUGAAtt-3' (123) | 898-916/846-864 |
| | antisens : 5'-UUCAUGCAGGCCCAGUGCUtt-3' (124) | |
| | | |
| siARN-NRP7+1 | sens : 5'-CACUGGGCCUGCAUGAAUUtt-3' (125) | 900-918/848-866 |
| | antisens : 5'-AAUUCAUGCAGGCCCAGUGtt-3' (126) | |
| | | |
| siARN-NRP7+2 | sens : 5'-ACUGGGCCUGCAUGAAUUCtt-3' (127) | 901-919/849-867 |
| | antisens : 5'-GAAUUCAUGCAGGCCCAGUtt-3' (128) | |
| | | |
| siARN-NRP1+2 | sens : 5'-AGUCUUACUGGUUUGAGAAtt-3' (129) | 480-498/428-446 |
| | antisens : 5'-UUCUCAAACCAGUAAGACUtc-3' (130) | |
| | | |
| siARN-NRP1+3 | sens : 5'-GUCUUACUGGUUUGAGAAUtt-3' (131) | 481-499/429-447 |
| | antisens : 5'-CCCGAACCAUCCGAACCUGtt-3' (132) | |
| | | |
| siARN-NM1-2 | sens : 5'-CAGGUUCGGAUGGUUCGGGtt-3' (133) | 478-496/427-444 |
| | antisens : 5'-UUCUCAAACCAGUAAGACUtc-3' (134) | |
| | | |
| siARN-NM1-1 | sens : 5'-AGGUUCGGAUGGUUCGGGAtt-3' (135) | 479-497/427-445 |
| | antisens : 5'-UCCCGAACCAUCCGAACCUtt-3' (136) | |
| | | |
| siARN-NM1+1 | sens : 5'-GUUCGGAUGGUUCGGGAACtt-3' (137) | 481-499/429-447 |
| | antisens : 5'-GUUCCCGAACCAUCCGAACtt-3' (138) | |

Des cellules VERO ont été transfectées avec différentes doses de ces siARN, puis infectées par le PPRV, RPV ou MV comme décrit dans la section « Matériel et Méthodes ».

3 à 5 jours après infection, la production des protéines N, M et F dans les cellules est mesurée par cytométrie en flux, comme décrit dans la section « Matériel et Méthodes ».

Les résultats obtenus sur près de 70 expérimentations sont illustrés dans le tableau IV ci-après.

**TABLEAU IV**

| Nature du siARN (concentration optimale : 100nM) | Inhibition de l'expression de la Nucléoprotéine (en %) | | | Inhibition de l'expression de la protéine de Matrice (en %) | | | Inhibition de l'expression de la protéine de Fusion (en %) | | |
|---|---|---|---|---|---|---|---|---|---|
| | PPRV | RPV | MV | PPRV | RPV | MV | PPRV | RPV | MV |
| NPPR1-3 | 7 | nt | nt | nt | nt | nt | nt | nt | nt |
| NPPR1-2 | 15 | nt | nt | nt | nt | nt | nt | nt | nt |
| NPPR1-1 | 78 | 0 | 0 | 72 | nt | nt | nt | nt | nt |
| NPPR1 | 90 | 0 | 0 | 89 | 0 | nt | nt | nt | nt |
| NPPR1+1 | 24 | nt | nt | nt | nt | nt | nt | nt | nt |
| NPPR1+2 | 28 | nt | nt | nt | nt | nt | nt | nt | nt |
| NPPR6-2 | 12 | nt | nt | nt | nt | nt | nt | nt | nt |
| NPPR6-1 | 35 | nt | nt | nt | nt | nt | nt | nt | nt |
| NPPR6 | 80 | 4 | nt | 85 | nt | nt | nt | nt | nt |
| NPPR6+1 | 56 | nt | nt | nt | nt | nt | nt | nt | nt |
| NPPR6+2 | 41 | nt | nt | nt | nt | nt | nt | nt | nt |
| NPPR7-2 | 35 | nt | nt | nt | nt | nt | nt | nt | nt |
| NPPR7-1 | 44 | nt | nt | nt | nt | nt | nt | nt | nt |
| NPPR7 | 87 | 0 | nt | 83 | nt | nt | nt | nt | nt |
| NPPR7+1 | 62,5 | nt | nt | nt | nt | nt | nt | nt | nt |
| NPPR7+2 | 41 | nt | nt | nt | nt | nt | nt | nt | nt |
| | | | | | | | | | |
| NRP1 | 0 | 35 | 0 | nt | 31 | nt | nt | nt | nt |
| NRP1+1 | 0 | 97 | 0 | nt | 92 | nt | nt | nt | nt |
| NRP1+2 | 0 | 70 | 0 | nt | 80 | nt | nt | nt | nt |
| NRP1+3 | nt | 45 | nt | nt | nt | nt | nt | nt | nt |
| NRP6-2 | nt | 11 | nt | nt | nt | nt | nt | nt | nt |
| NRP6-1 | nt | 0 | nt | nt | nt | nt | nt | nt | nt |
| NRP6 (50nM) | nt | 85 | nt | 5 | nt | nt | nt | 83 | nt |
| NRP6+1 | nt | 36 | nt | nt | nt | nt | nt | nt | nt |
| NRP6+2 | nt | 41 | nt | nt | nt | nt | nt | nt | nt |
| NRP7-2 | nt | 18 | nt | nt | nt | nt | nt | nt | nt |
| NRP7-1 | nt | 36 | nt | nt | nt | nt | nt | nt | nt |
| NRP7 | nt | 76,5 | nt | nt | nt | nt | nt | 81 | nt |
| NRP7+1 | nt | 85 | nt | 5 | nt | nt | nt | 86 | nt |
| NRP7+2 | nt | 49 | nt | nt | nt | nt | nt | nt | nt |
| | | | | | | | | | |
| NM1-2 | nt | nt | 35 | nt | nt | nt | nt | nt | nt |
| NM1-1 | nt | 0 | 85 | nt | nt | nt | nt | nt | nt |
| NM1 | 0 | 0 | 90 | nt | nt | nt | nt | nt | nt |
| NM1+1 | nt | nt | 28 | nt | nt | nt | nt | nt | nt |

| | | | | | | | | | |
|---|---|---|---|---|---|---|---|---|---|
| nt : non testé. | | | | | | | | | |

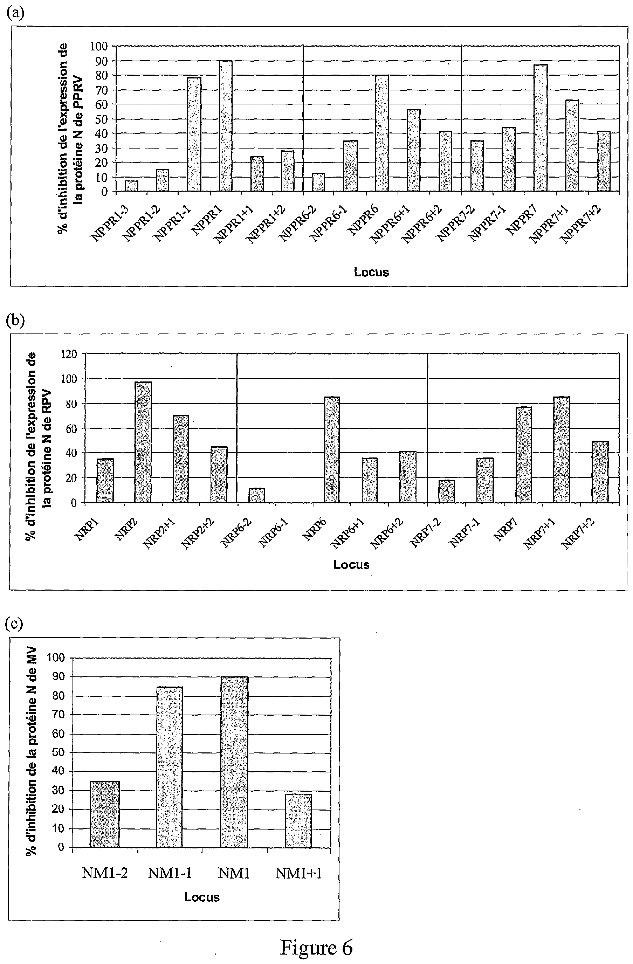

Le pourcentage d'inhibition de la production de la protéine N virale induit par la transfection des différents siARN dans des cellules VERO est également illustré sur la figure 6 (a) pour le virus PPRV, (b) pour le virus RPV et (c)pour le virus MV.

### Inhibition de l'expression de la protéine N :

### NPPRV:

On observe une inhibition de 90% de l'expression de la protéine N avec le siARN NPPR1. Cette inhibition diminue à 78%, 15% et 7% avec respectivement NPPR1-1, NPPR1-2 et NPPR1-3 et à moins de 30% avec NPPR1+1 et NPPR1+2. On observe de même 80% d'inhibition avec le siARN NPPR6, 56% d'inhibition avec NPPR6+1 et de 41% d'inhibition avec NPPR6+2. Avec NPPR6-1 l'inhibition est de 35% et de 70% avec NPPR6-2. Avec NPPR7, on observe 87% d'inhibition de l'expression de la protéine N, et avec NPPR7-1 et NPPR7-2 on observe respectivement 44% et 35% d'inhibition. Avec NPPR7+1, le pourcentage d'inhibition est de 62.5% et de 41% avec NPPR7+2. Les résultats obtenus avec le siARN-NPPR1 confirment ceux obtenus précédemment à l'exemple 3 (Tableau II). En ce qui concerne les siARNs NPPR6 et NPPR7, les résultats obtenus à partir d'expérimentations répétées montrent que leur efficacité est supérieure à celle qui avait été évaluée à l'Exemple 3 à partir d'une seule expérimentation.

### NRPV :

On observe une inhibition de 35% de l'expression de la protéine N avec le siARN NRP1 et 97% avec NRP1+1. Cette inhibition diminue à respectivement 70% et 45% avec NRP1+2 et NRP1+3. On observe 86% d'inhibition avec le siARN NRP6. Cette inhibition diminue à 11% avec NRP6-2, à 36% et 41% avec respectivement NRP6+1 et NRP6+2, et on n'observe aucune inhibition avec NRP6-1. On observe 76.5% d'inhibition de l'expression de la protéine N avec NRP7, 36% et 18% d'inhibition pour respectivement NRP7-1 et NRP7-2. Avec NRP7+1, le pourcentage d'inhibition est de 85%, et de 49% pourNRP7+2.

### MV :

On observe une inhibition de 90% de l'expression de la protéine N pour NM1. Cette inhibition diminue à 85%, et 35% avec respectivement NM1-1 et NM1-2 et à moins de 30% avec NM1+1.

### Inhibition de l'expression de la protéine M :

Chez PPRV, on observe une diminution de l'expression de 72%, 89%, 85% et 83 avec respectivement NPPR1-1, NPPR1, NPPR6 et NPPR7. Chez RPV, la diminution de l'expression est de 31%, 92% et 80% avec respectivement NRP1, NRP1+1 et NRP1+2.

### Inhibition de l'expression de la protéine F :

Chez RPV, l'inhibition de l'expression est de 83%, 81% et 86% avec respectivement NRP6, NRP7 et NRP7+1.

Ces résultats montrent que les loci reconnus par les siARN NPPR1, NPPR6, NPPR7, NRP1+1, NRP6 et NM1 constituent une cible particulièrement intéressante et très précise, pour l'extinction du gène N de morbillivirus. Dans le cas du siARN NRP7, le pourcentage d'inhibition le plus important est obtenu pour NRP7+1, le locus efficace est donc décalé d'une base en 3'. La mesure de l'expression des protéines M et F, montre que l'inhibition de la protéine N entraine également une inhibition très fortes des autres protéines virales.

## Revendications

1. ARN interférent, capable d'inhiber l'expression du gène N codant pour la nucléoprotéine d'un morbillivirus, **caractérisé en ce qu'**il comprend une séquence générale UYYCNAACC ANYNNRWYB (SEQ ID NO: 10) et qu'il est dirigé contre une région de l'ARNm du gène *N* codant pour la nucléoprotéine d'un morbillivirus, ladite région contenant un motif cible défini par la séquence générale suivante :
VRWYNNRNUGGUUNGRRA (SEQ ID NO: 1)
où A = Adénine ; C = Cytosine ; G = Guanine ; U = Uracile ; K = G ou U ; M = A ou C; R = A ou G; S = G ou C; Y = C ou U; W = A ou U; B = G, U ou C; D = G, A ou U ; H = A ou C ou U; V = G, A ou C ; N = A ou C ou G ou U.

2. ARN interférent selon la revendication 1, capable d'inhiber l'expression du gène N codant pour la nucléoprotéine d'un morbillivirus, **caractérisé en ce qu'**il comprend l'une des séquences générales suivantes : UYUCDAACCADYNNRWYY (SEQ ID NO: 11) ou UYYCNAACC ANYNNRWYBB (SEQ ID NO: 12) et que ledit motif cible est défini par l'une des séquences générales suivantes :
RRWYNNRHUGGUUHGARA (SEQ ID NO: 2)
ou
VVRWYNNRNUGGUUNGRRA (SEQ ID NO: 3)
où A = Adénine ; C = Cytosine ; G = Guanine ; U = Uracile ; K = G ou U; M = A ou C; R = A ou G; S = G ou C; Y = C ou U; W = A ou U; B = G, U ou C; D = G, A ou U ; H = A ou C ou U; V = G, A ou C ; N = A ou C ou G ou U.

3. ARN interférent, capable d'inhiber l'expression du gène N codant pour la nucléoprotéine d'un morbillivirus, **caractérisé en ce qu'**il comprend une séquence générale ARNGMNACCAWRAAYMKNC (SEQ ID NO: 37) et qu'il est dirigé contre une région de l'ARNm du gène *N* codant pour la nucléoprotéine d'un morbillivirus, ladite région contenant un motif cible défini par la séquence générale suivante :
GNMKRUUYWUGGUNKCNYU (SEQ ID NO: 19)
où A = Adénine ; C = Cytosine ; G = Guanine ; U = Uracile ; K = G ou U; M = A ou C; R = A ou G; S = G ou C; y = C ou U; W = A ou U; B = G, U ou C; D = G, A ou U ; H = A ou C ou U; V = G, A ou C ; N = A ou C ou G ou U.

4. ARN interférent, capable d'inhiber l'expression du gène N codant pour la nucléoprotéine d'un morbillivirus, **caractérisé en ce qu'**il comprend une séquence générale AYUCRUGHARNSCNARDG (SEQ ID NO: 39) et qu'il est dirigé contre une région de l'ARNm du gène *N* codant pour la nucléoprotéine d'un morbillivirus, ladite région contenant un motif cible défini par la séquence générale suivante :
CHYUNGSNYUDCAYGARU (SEQ ID NO: 21)
où A = Adénine ; C = Cytosine ; G = Guanine ; U = Uracile ; K = G ou U; M = A ou C; R = A ou G; S = G ou C; y = C ou U; W = A ou U; B = G, U ou C; D = G, A ou U ; H = A ou C ou U; V = G, A ou C ; N = A ou C ou G ou U.

5. ARN interférent selon la revendication 4, capable d'inhiber l'expression du gène N codant pour la nucléoprotéine d'un morbillivirus, **caractérisé en ce qu'**il comprend l'une des séquences générales suivantes : AAYUCRUGHARNS CNARDG (SEQ ID NO: 40) ou AYUCRUGHARNSCNARDGC (SEQ ID NO: 41) et que ledit motif cible est défini par l'une des séquences générales suivantes :
- CHYUNGSNYUDCAYGARUU (SEQ ID NO: 22) ;
- GCHYUNGSNYUDCAYGARU (SEQ ID NO: 23)
où A = Adénine ; C = Cytosine ; G = Guanine ; U = Uracile ; K = G ou U; M = A ou C ; R = A ou G; S = G ou C ; Y = C ou U; W = A ou U ; B = G, U ou C ; D = G, A ou U ; H = A ou C ou U; V = G, A ou C ; N = A ou C ou G ou U.

6. ARN interférent selon une quelconque des revendications 1 à 5, **caractérisé en ce qu'**il est choisi parmi les siARNs, les shARNs et les pre-miARNs.

7. Vecteur d'expression contenant une séquence d'ADN pouvant être transcrite en un ARN interférent selon une quelconque des revendications 1 à 6, placée sous contrôle d'un promoteur approprié, ledit vecteur étant capable d'exprimer l'ARN interférent selon une quelconque des revendications 1 à 6.

8. Utilisation d'un ARN interférent selon une quelconque des revendications 1 à 6 pour l'obtention d'un médicament destiné au traitement ou à la prévention d'une infection à morbillivirus.

9. Utilisation selon la revendication 8, **caractérisée en ce que** l'on utilise une combinaison d'au moins 2 ARN interférents choisis parmi :
- un ARN interférent selon une quelconque des revendications 1 ou 2 ;
- un ARN interférent selon la revendication 3 ;
- un ARN interférent selon une quelconque des revendications 4 ou 5.

10. Utilisation selon une quelconque des revendications 8 ou 9, **caractérisée en ce que** ledit morbillivirus est choisi dans le groupe constitué du virus de la rougeole (MV), du virus de la peste bovine (RPV), du virus de la peste des petits ruminants (PPRV), du virus de la maladie de Carré (CDV), du virus de la peste des phoques (PDV), et du morbillivirus du dauphin (DMV).

11. Composition pharmaceutique comprenant un ARN interférent selon une quelconque des revendications 1 à 6.

12. Composition pharmaceutique selon la revendication 11, **caractérisée en ce qu'**elle comprend une combinaison d'au moins 2 ARN interférents choisis parmi :
- un ARN interférent selon une quelconque des revendications 1 ou 2 ;
- un ARN interférent selon la revendication 3 ;
- un ARN interférent selon une quelconque des revendications 4 ou 5.

13. Composition pharmaceutique selon une quelconque des revendications 11 ou 12, **caractérisée en ce qu'**il s'agit d'un vaccin.

## Patentansprüche

1. Interferierende RNA, die imstande ist, die Expression des für das Nukleoprotein eines Morbillivirus codierenden Gens N zu hemmen, **dadurch gekennzeichnet, dass** sie eine allgemeine Sequenz UYYCNAACC ANYNNRWYB (SEQ ID NR. 10) umfasst und dass sie gegen eine Region der mRNA des Gens *N* gerichtet ist, das für das Nukleoprotein eines Morbillivirus codiert, wobei die Region ein Zielmotiv enthält, das durch die folgende allgemeine Sequenz bestimmt ist:
VRWYNNRNUGGUUNGRRA (SEQ ID NR. 1),
wobei A = Adenin; C = Cytosin; G = Guanin; U = Uracil; K = G oder U; M = A oder C; R = A oder G; S = G oder C; Y = C oder U; W = A oder U; B = G, U oder C; D = G, A oder U; H = A oder C oder; V = G, A oder C; N = A oder C oder G oder U ist.

2. Interferierende RNA nach Anspruch 1, die imstande ist, die Expression des für das Nukleoprotein eines Morbillivirus codierenden Gens N zu hemmen, **dadurch gekennzeichnet, dass** sie eine der folgenden allgemeinen Sequenzen UYUCDAACCADYNNRWYY (SEQ ID NR. 11) oder UYYCNAACC ANYNNRWYBB (SEQ ID NR. 12) umfasst und dass das Zielmotiv von einer der folgenden allgemeinen Sequenzen bestimmt ist:
RRWYNNRHUGGUUHGARA (SEQ ID NR. 2)
oder
VVRWYNNRNUGGUUNGRRA (SEQ ID NR. 3)
wobei A = Adenin; C = Cytosin; G = Guanin; U = Uracil; K = G oder U; M = A oder C; R = A oder G; S = G oder C; Y = C oder U; W = A oder U; B = G, U oder C; D = G, A oder U; H = A oder C oder U; V = G, A oder C; N = A oder C oder G oder U ist.

3. Interferierende RNA, die imstande ist, die Expression des für das Nukleoprotein eines Morbillivirus codierenden Gens N zu hemmen, **dadurch gekennzeichnet, dass** sie eine allgemeine Sequenz ARNGMNACCAWRAAYMKNC (SEQ ID NR. 37) umfasst und dass sie gegen eine Region der mRNA des Gens *N* gerichtet ist, das für das Nukleoprotein eines Morbillivirus codiert, wobei die Region ein Zielmotiv enthält, das durch die folgende allgemeine Sequenz bestimmt ist:
GNMKRUUYWUGGUNKCNYU (SEQ ID NR. 19),
wobei A = Adenin; C = Cytosin; G = Guanin; U = Uracil; K = G oder U; M = A oder C; R = A oder G; S = G oder C; Y = C oder U; W = A oder U; B = G, U oder C; D = G, A oder U; H = A oder C oder U; V = G, A oder C; N = A oder C oder G oder U ist.

4. Interferierende RNA, die imstande ist, die Expression des für das Nukleoprotein eines Morbillivirus codierenden Gens N zu hemmen, **dadurch gekennzeichnet, dass** sie eine allgemeine Sequenz AYUCRUGHARNSCNARDG (SEQ ID NR. 39) umfasst und dass sie gegen eine Region der mRNA des Gens *N* gerichtet ist, das für das Nukleoprotein eines Morbillivirus codiert, wobei die Region ein Zielmotiv enthält, das durch die folgende allgemeine Sequenz bestimmt ist:
CHYUNGSNYUDCAYGARU (SEQ ID NR. 21),
wobei A = Adenin; C = Cytosin; G = Guanin; U = Uracil; K = G oder U; M = A oder C; R = A oder G; S = G oder C; Y = C oder U; W = A oder U; B = G, U oder C; D = G, A oder U; H = A oder C oder U; V = G, A oder C; N = A oder C oder G oder U ist.

5. Interferierende RNA nach Anspruch 4, die imstande ist, die Expression des für das Nukleoprotein eines Morbillivirus codierenden Gens N zu hemmen, **dadurch gekennzeichnet, dass** sie eine der folgenden allgemeinen Sequenzen AAYUCRUGHARNSCNARDG (SEQ ID NR. 40) oder AYUCRUGHARNSCNARDGC (SEQ ID NR. 41) umfasst und dass das Zielmotiv von einer der folgenden allgemeinen Sequenzen bestimmt ist:
- CHYUNGSNYUDCAYGARUU (SEQ ID NR. 22),
- GCHYUNGSNYUDCAYGARU (SEQ ID NR. 23),
wobei A = Adenin; C = Cytosin; G = Guanin; U = Uracil; K = G oder; M = A oder C; R = A oder G; S = G oder C; Y = C oder U; W = A oder U; B = G, U oder C; D = G, A oder U; H = A oder C oder U; V = G, A oder C; N = A oder C oder G oder U ist.

6. Interferierende RNA nach einem der Ansprüche 1 bis 5, **dadurch gekennzeichnet, dass** sie aus den siRNA, den shRNA und den pre-miRNA ausgewählt ist.

7. Expressionsvektor, enthaltend eine DNA-Sequenz, die in eine interferierende RNA nach einem der Ansprüche 1 bis 6 transkribierbar ist, platziert unter der Kontrolle eines geeigneten Promoters, wobei der Vektor imstande ist, die interferierende RNA nach einem der Ansprüche 1 bis 6 zu exprimieren.

8. Verwendung einer interferierenden RNA nach einem der Ansprüche 1 bis 6 für den Erhalt eines Arzneimittels, das zur Behandlung oder Vorbeugung einer Infektion mit Morbillivirus bestimmt ist.

9. Verwendung nach Anspruch 8, **dadurch gekennzeichnet, dass** eine Kombination aus mindestens zwei interferierenden RNA verwendet wird, die ausgewählt sind aus:
- einer interferierenden RNA nach einem der Ansprüche 1 oder 2,
- einer interferierende RNA nach Anspruch 3,
- einer interferierende RNA nach einem der Ansprüche 4 oder 5.

10. Verwendung nach einem der Ansprüche 8 oder 9, **dadurch gekennzeichnet, dass** das Morbillivirus aus der Gruppe ausgewählt ist, die von dem Masernvirus (MV), dem Rinderpestvirus (RPV), dem Pseudorinderpestvirus (PPRV), dem Staupevirus (CDV), dem Robbenpestvirus (PDV) und dem Delphin-Morbillivirus (DMV) gebildet ist.

11. Pharmazeutische Zusammensetzung, umfassend eine interferierende RNA nach einem der Ansprüche 1 bis 6.

12. Pharmazeutische Zusammensetzung nach Anspruch 11, **dadurch gekennzeichnet, dass** sie eine Kombination aus mindestens zwei interferierenden RNA umfasst, die ausgewählt sind aus:
- einer interferierenden RNA nach einem der Ansprüche 1 oder 2,
- einer interferierende RNA nach Anspruch 3,
- einer interferierende RNA nach einem der Ansprüche 4 oder 5.

13. Pharmazeutische Zusammensetzung nach einem der Ansprüche 11 oder 12, **dadurch gekennzeichnet, dass** es sich um einen Impfstoff handelt.

## Claims

1. Interfering RNA, able to inhibit the expression of the N gene encoding the nucleoprotein of a morbillivirus, **characterised in that** it comprises a general sequence UYYCNAACC ANYNNRWYB (SEQ ID NO: 10) and that it is directed against a region of mRNA of the N gene encoding the nucleoprotein of a morbillivirus, said region containing a target motif defined by the following general sequence:
VRWYNNRNUGGUUNGRRA (SEQ ID NO: 1)
where A = Adenine; C = Cytosine; G = Guanine; U = Uracil; K = G or U; M = A or C; R = A or G; S = G or C; Y = C or U; W = A or U; B = G, U or C; D = G, A or U; H = A or C or U; V = G, A or C; N = A or C or G or U.

2. Interfering RNA according to claim 1, able to inhibit the expression of the N gene encoding the nucleoprotein of a morbillivirus, **characterised in that** it comprises one of the following general sequences: UYUCDAACCADYNNRWYY (SEQ ID NO: 11) or UYYCNAACC ANYNNRWYBB (SEQ ID NO: 12) and that said target motif is defined by one of the following general sequences:
RRWYNNRHUGGUUHGARA (SEQ ID NO: 2) or
VVRWYNNRNUGGUUNGRRA (SEQ ID NO: 3)
where A = Adenine; C = Cytosine; G = Guanine; U = Uracil; K = G or U; M = A or C; R = A or G; S = G or C; Y = C or U; W = A or U; B = G, U or C; D = G, A or U; H = A or C or U; V = G, A or C; N = A or C or G or U.

3. Interfering RNA, able to inhibit the expression of the N gene encoding the nucleoprotein of a morbillivirus, **characterised in that** it comprises a general sequence ARNGMNACCA WRAAYMKNC (SEQ ID NO: 37) and that it is directed against a region of mRNA of the N gene encoding the nucleoprotein of a morbillivirus, said region containing a target motif defined by the following general sequence:
GNMKRUUYWUGGUNKCNYU (SEQ ID NO : 19)
where A = Adenine; C = Cytosine; G = Guanine; U = Uracil; K = G or U; M = A or C; R = A or G; S = G or C; Y = C or U; W = A or U; B = G, U or C; D = G, A or U; H = A or C or U; V = G, A or C; N = A or C or G or U.

4. Interfering RNA, able to inhibit the expression of the N gene encoding the nucleoprotein of a morbillivirus, **characterised in that** it comprises a general sequence AYUCRUGHARNSCNARDG (SEQ ID NO: 39) and that it is directed against a region of mRNA of the N gene encoding the nucleoprotein of a morbillivirus, said region containing a target motif defined by the following general sequence:
CHYUNGSNYUDCAYGARU (SEQ ID NO: 21)
where A = Adenine; C = Cytosine; G = Guanine; U = Uracil; K = G or U; M = A or C; R = A or G; S = G or C; Y = C or U; W = A or U; B = G, U or C; D = G, A or U; H = A or C or U; V = G, A or C; N = A or C or G or U.

5. Interfering RNA according to claim 4, able to inhibit the expression of the N gene encoding the nucleoprotein of a morbillivirus, **characterised in that** it comprises one of the following general sequences: AAYUCRUGHARNS CNARDG (SEQ ID NO: 40) or AYUCRUGHARNSCNARDGC (SEQ ID NO: 41) and that said target motif is defined by one of the following general sequences:
- CHYUNGSNYUDCAYGARUU (SEQ ID NO: 22);
- GCHYUNGSNYUDCAYGARU (SEQ ID NO: 23)
where A = Adenine; C = Cytosine; G = Guanine; U = Uracil; K = G or U; M = A or C; R = A or G; S = G or C; Y = C or U; W = A or U; B = G, U or C; D = G, A or U; H = A or C or U; V = G, A or C; N = A or C or G or U.

6. Interfering RNA according to any one of claims 1 to 5, **characterised in that** it is selected from the siRNAs, shRNAs and pre-miRNAs.

7. Expression vector containing a DNA sequence able to be transcribed into an interfering RNA according to any one of claims 1 to 6, placed under control of an appropriate promoter, said vector able to express the interfering RNA according to any one of claims 1 to 6.

8. Use of an interfering RNA according to any one of claims 1 to 6 for the obtaining of a drug intended for treating or preventing morbillivirus infection.

9. Use according to claim 8, **characterised in that** a combination of at least 2 interfering RNAs is used selected from:
- an interfering RNA according to any one of claims 1 or 2;
- an interfering RNA according to claim 3;
- an interfering RNA according to any one of claims 4 or 5.

10. Utilisation according to any one of claims 8 or 9, **characterised in that** said morbillivirus is selected from the group consisting of measles virus (MV), rinderpest virus (RPV), small ruminants virus (PPRV), Carré disease virus (CDV), phocine distemper virus (PDV), and dolphin morbillivirus (DMV).

11. Pharmaceutical composition comprising an interfering RNA according to any one of claims 1 to 6.

12. Pharmaceutical composition according to claim 11, **characterised in that** it comprises a combination of at least 2 interfering RNAs selected from;
- an interfering RNA according to any one of claims 1 or 2;
- an interfering RNA according to claim 3;
- an interfering RNA according to any one of claims 4 or 5.

13. Pharmaceutical composition according to any one of claims 11 or 12, **characterised in that** it is a vaccine.
